# EUROPEAN PATENT SPECIFICATION

(11) **EP 1 176 618 B1**
(45) Date of publication and mention of the grant of the patent: **18.02.2004**
(21) Application number: 01117952.0
(22) Date of filing: 24.07.2001
(51) Int. Cl.: H01G 9/20, C07D 213/22, C07F 15/00, C09B 57/10

(54) **Ruthenium complex dye, photoelectric conversion device and photoelectric cell**
Ruthenium-Komplexfarbstoff, photoelektrische Umwandlungsvorrichtung und photoelektrische Zelle
Colorant de complexes de ruthénium, dispositif de conversion photoélectrique et cellule photoélectrochimique

(30) Priority: 25.07.2000 JP 2000224635; 15.02.2001 JP 2001038421
(43) Date of publication of application: 30.01.2002
(73) Proprietor: FUJI PHOTO FILM CO., LTD., Kanagawa-ken (JP)
(72) Inventor: Watanabe, Tetsuya, Minami-Ashigara-shi, Kanagawa-ken (JP); Tsukahara, Jiro, Minami-Ashigara-shi, Kanagawa-ken (JP)
(74) Representative: Solf, Alexander, Dr.

(56) References cited:
- WO-A-98/50393
- WO-A-99/55675
- US-A- 3 810 848
- US-A- 4 424 359
- US-A- 5 399 694
- YANG ET AL: "Photoelectric performance study of nanocrystalling TiO2 film sensitized by phenylphosphonated polypyridyl ruthenium complex" CHEMICAL ABSTRACTS + INDEXES, AMERICAN CHEMICAL SOCIETY. COLUMBUS, US, vol. 11, no. 128, 16 March 1998 (1998-03-16), XP002067003 ISSN: 0009-2258 & GANGUANG KEXUE YU GUANG HUAXUE, vol. 15, no. 4, 1997, page 293-296

## Description

### BACKGROUND OF THE INVENTION

The present invention relates to a photoelectric conversion device and a photoelectric cell comprising a semiconductor fine particle sensitized by a ruthenium complex dye.

To put solar power generation into practical use, solar cells comprising monocrystalline silicon, polycrystalline silicon, amorphous silicon, a compound such as cadmium telluride and indium copper selenide, etc. have been mainly researched and developed. However, for a widespread use of the solar cells as a home power source, etc., such problems as a high production cost, difficulty in security of raw materials and a long energy payback time must be overcome. Although a variety of solar cells using an organic material are also proposed with the objects of increasing the surface area and reducing costs of the cell, such solar cells have disadvantages of low conversion efficiency and poor durability.

Under such circumstances, Nature, Vol. 353, Page 737 to 740 (1991), United States Patent No. 4,927,721, WO 94/04497, etc. disclosed photoelectric conversion devices and solar cells comprising a semiconductor fine particle sensitized by a dye, and materials and production methods therefor. The solar cells are a wet-type solar cell using a titanium dioxide porous thin film spectrally sensitized by a ruthenium complex dye as a working electrode. The primary advantage for the wet-type solar cells is that the solar cell can be produced with low costs because it can use an inexpensive metal oxide semiconductor such as titanium dioxide without purification to a high purity. The secondary advantage therefor is that the solar cell can convert light in almost entire visible wavelength region to electricity because the dye used therein has a broad absorption spectrum.

However, ruthenium complex dyes used for the wet-type solar cell is remarkably limited with respect to the structure as compared with sensitizing dyes for photography, and the ruthenium complex dyes are demanded to be structurally changed correspondingly to desired properties. Further, as disclosed in WO 98/50393, etc., although the known ruthenium complex dyes can utilize light in almost entire visible wavelength region, they can hardly utilize infrared light contained in solar light abundantly, particularly such that has a wavelength of 800 nm or more.

The Chemical Abstract, 128, (no. 11), 772 (1998) provides a photolectric performance of nanocrystalling TiO₂ film sensitized by a phenylphosphonated polypyridyl ruthenium complex.

Furthermore, the WO 99 55 675 A describes a method for producing dipyridyls with different substituents comprising alkyl, alkoxy, acyloxy, aryloxy, perfluoroacyloxy and much more.

### OBJECT AND SUMMARY OF THE INVENTION

An object of the present invention is to provide a photoelectric conversion device and a photoelectric cell comprising a semiconductor fine particle sensitized by a ruthenium complex dye, which is excellent in photoelectric conversion efficiency.

Another object of the present invention is to provide a ruthenium complex dye used for the photoelectric conversion device and the photoelectric cell, which can efficiently sensitize the semiconductor fine particle and utilize a light having a wavelength of 800 nm or more.

As a result of intense research in view of the above objects, the inventor has found that a photoelectric conversion device, which uses a semiconductor fine particle sensitized by a ruthenium complex dye comprising a particular ligand, can convert a light having a wavelength of 800 nm or more to electricity, to be excellent in photoelectric conversion efficiency. The present invention has been accomplished by the finding.

Thus, a photoelectric conversion device of the present invention comprises a semiconductor fine particle sensitized by a ruthenium complex dye is represented by the following general formula (IV) 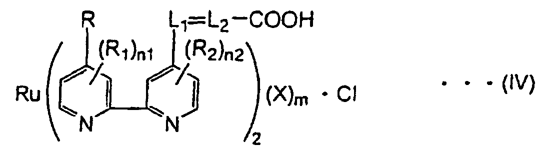 wherein *R, R*_{*1*} and *R*_{*2*} independently represent a substituent; *n1* and *n2* independently represent an integer of 0 to 2; *R*_{*1*}*'s* and *R*_{*2*}*'s* are the same or different groups that optionally bond together to form a ring when *n1* and *n2* are 2, respectively; *L*_{*1*} and *L*_{*2*} independently represent a substituted or unsubstituted methine group; *X* represents a monodentate or bidentate ligand; *m* represents 1 or 2; and *CI* represents a counter ion optionally comprised to neutralize charge of the ruthenium complex dye.

A photoelectric cell of the present invention comprises the above-mentioned photoelectric conversion device of the present invention.

With respect to the photoelectric conversion device and the photoelectric cell of the present invention, the photoelectric conversion efficiency is further improved by satisfying any of the following conditions (1) to (3)
(1) *m* in the general formula (IV) is particularly preferably 1.
(2) *X* in the general formula (IV) is particularly preferably a ligand that coordinates to the transition metal atom via an isothiocyanate group and/or an isocyanate group.
(3) The semiconductor fine particle is preferably a titanium oxide fine particle.

### BRIEF DESCRIPTION OF THE DRAWINGS

Fig. 1 is a partial cross sectional view showing a preferable structure of a photoelectric conversion device of the present invention;
Fig. 2 is a partial cross sectional view showing a preferable structure of a photoelectric conversion device of the present invention;
Fig. 3 is a partial cross sectional view showing a preferable structure of a photoelectric conversion device of the present invention;
Fig. 4 is a partial cross sectional view showing a preferable structure of a photoelectric conversion device of the present invention;
Fig. 5 is a partial cross sectional view showing a preferable structure of a photoelectric conversion device of the present invention;
Fig. 6 is a partial cross sectional view showing a preferable structure of a photoelectric conversion device of the present invention;
Fig. 7 is a partial cross sectional view showing a preferable structure of a photoelectric conversion device of the present invention;
Fig. 8 is a partial cross sectional view showing a preferable structure of a photoelectric conversion device of the present invention;
Fig. 9 is a partial cross sectional view showing a preferable structure of a photoelectric conversion device of the present invention; and
Fig. 10 is a partial cross sectional view showing a structure of the photoelectric conversion device according to the EXAMPLES.

### DETAILED DESCRIPTION OF THE PREFERRED EMBODIMENTS

### [1] Ruthenium Complex Dye

A ruthenium complex dye used in this invention will be described in detail below. Incidentally, when the ruthenium complex dye contains an alkyl group, an alkenyl group, an alkynyl group, an alkylene group, etc., these groups may have a straight or branched structure and may be substituted or unsubstituted. Further, when the ruthenium complex dye contains an aryl group, a heterocyclic group, a cycloalkyl group, etc., these groups may have a monocyclic structure or a polycyclic structure such as a condensed ring and a ring assemblage, and may be substituted or unsubstituted.

The ruthenium complex dye used in this invention is represented by the following general formula (IV)

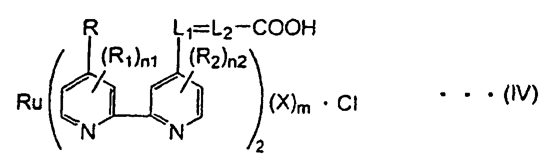

In the general formula (IV), *R*_{*1*}, *R*_{*2*} and *R*_{*3*} independently represent a substituent. Examples of the substituent include alkyl groups, alkenyl groups, cycloalkyl groups, aryl groups, heterocyclic groups, alkoxy groups, a hydroxyl group, amino groups, halogen atoms, etc. Among them, alkyl groups are preferable. *n1* and *n2* independently represent an integer of 0 to 2. *R*_{*1*}*'s* and *R*_{*2*}*'s* are the same or different groups that optionally bond together to form a ring when *n1* and *n2* are 2; respectively.

In the general formula (IV), *L*_{*1*} and *L*_{*2*} independently represent a substituted or unsubstituted methine group. Examples of a substituent on the methine group include alkyl groups, alkenyl groups, cycloalkyl groups, aryl groups, heterocyclic groups, alkoxy groups, amino groups, halogen atoms, etc. It is preferable that *L*_{*1*} and *L*_{*2*} are an unsubstituted methine group or a methine group substituted by an alkyl group, respectively.

Specific examples *L-1* to *L-10* and *L-17* to *L-27* of the ligand represented by the general formula (IV) will be illustrated below without intention of restricting the scope of the present invention defined by the claims attached hereto. Incidentally, a proton of a group such as -COOH, -SO₃H, etc. contained in the specific examples is optionally dissociated.

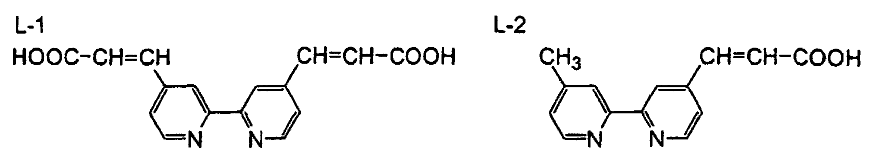

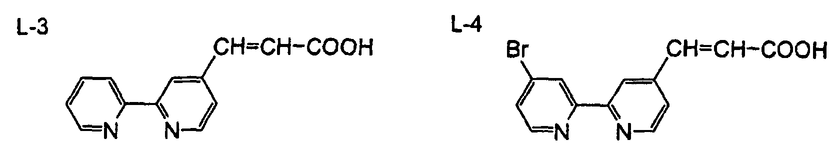

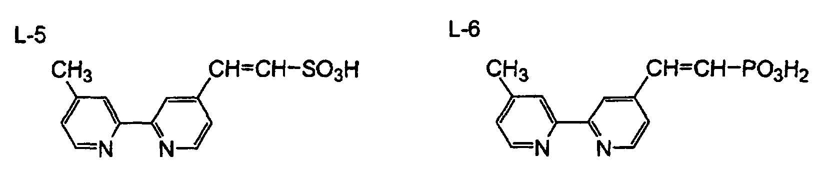

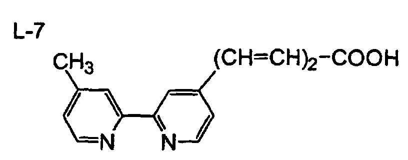

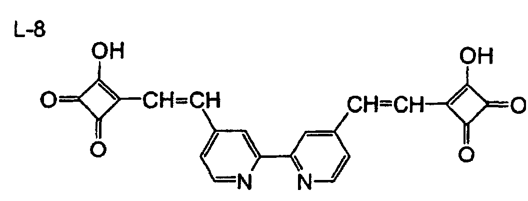

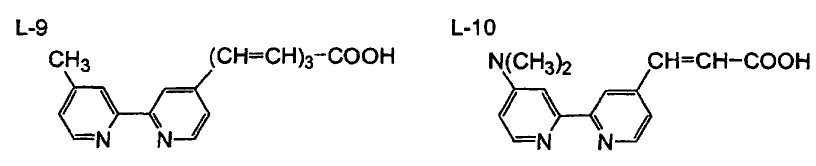

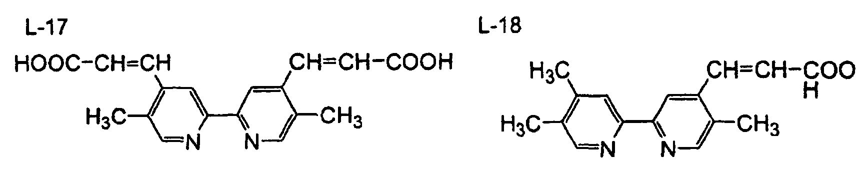

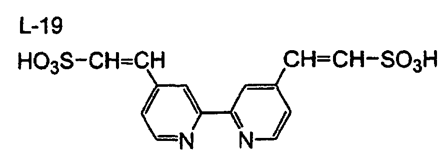

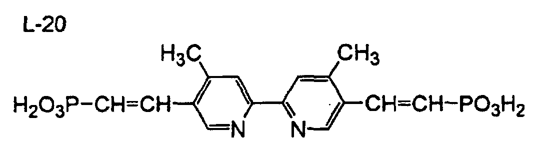

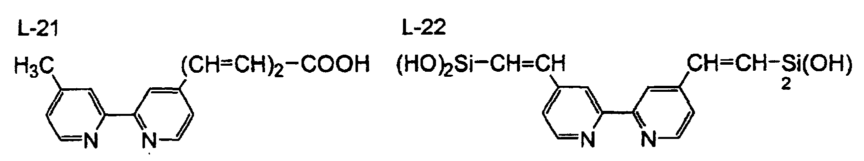

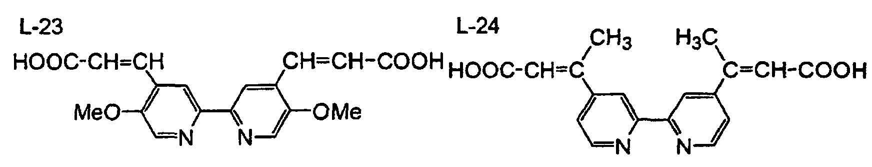

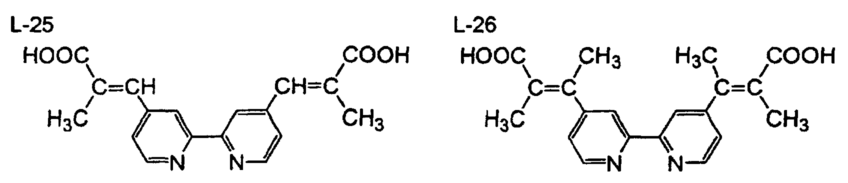

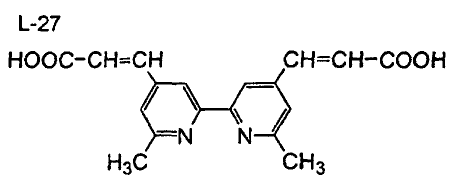

The metal complex dye comprising a ligand represented by the general formula (I) is preferably represented by the following general formula (III):

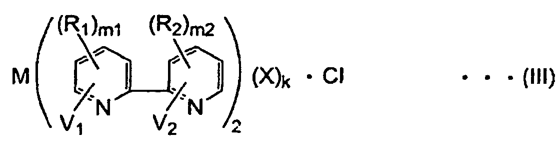

In the general formula (III), *V*_{*1*}*, V*_{*2*}*, R*_{*1*}*, R*_{*2*}*, m1* and *m2* are the same as those in the general formula (I), respectively.

### (1) Transition Metal Atom M

In the general formula (IV), *M* represents a transition metal atom. *M* is preferably a transition metal atom that can form four- or six-coordinated complex, more preferably Ru, Fe, Os, Cu, W, Cr, Mo, Ni, Pd, Pt, Co, Ir, Rh, Re, Mn or Zn, furthermore preferably Ru, Fe, Os or Cu, particularly preferably Ru.

### (2) Ligand X

In the general formula (IV), *X* represents a monodentate or bidentate ligand, and *k* indicating the number *of X* is 1 or 2.

Examples of the ligand represented by X include monodentate or bidentate ligands that coordinates to the transition metal atom via one or two group selected from the group consisting of: acyloxy groups preferably having 1 to 20 carbon atom such as an acetyloxy group, a benzoyloxy group and a oxalylene group (-OC(O)C(O)O-); acylthio groups preferably having 1 to 20 carbon atom such as an acetylthio group and a benzoylthio group; acylaminooxy groups preferably having 1 to 20 carbon atom such as an *N*-methylbenzoylaminooxy group (PhC(O)N(CH₃)O-) and an acetylaminooxy group (CH₃C(O)NHO-); thioacyloxy groups preferably having 1 to 20 carbon atom such as a thioacetyloxy group (CH₃C(S)O-); thioacylthio groups preferably having 1 to 20 carbon atom such as a thioacetylthio group (CH₃C(S)S-) and a thiobezoylthio group (PhC(S)S-); thiocarbonate groups preferably having 1 to 20 carbon atom such as an ethylthiocarbonate group and a phenylthiocarbonate group; dithiocarbonate groups preferably having 1 to 20 carbon atom such as an ethyldithiocarbonate group (C₂H₅OC(S)S-); trithiocarbonate groups preferably having 1 to 20 carbon atom such as an ethyltrithiocarbonate group (C₂H₅SC(S)S-); alkylthio groups preferably having 1 to 20 carbon atom such as a methylthio group and an ethylenedithio group; arylthio groups preferably having 6 to 20 carbon atoms such as a benzenethio group and a 1,2-phenylenedithio group; alkoxy groups preferably having 1 to 20 carbon atom such as a methoxy group and an ethylenedioxy group; aryloxy groups preferably having 6 to 20 carbon atoms such as a phenoxy group and a 1,2-phenylenedioxy group; an isothiocyanate group; an isocyanate group; a cyanate group; and a thiocyanate group.

Examples of the ligand represented by *X* further include: β-diketonato ligands preferably having 3 to 20 carbon atoms such as CH₃C(O···)CH=C(O-) CH₃; β-dithioketonato ligands preferably having 3 to 20 carbon atoms such as CH₃C(S···)CH=C(S-)CH₃); β-ketothionato ligands preferably having 3 to 20 carbon atoms such as CH₃C(O···)CH=C(S-)CH₃); β-thionketonato ligands preferably having 3 to 20 carbon atoms such as CH₃C(S···)CH=C(O-)CH₃); dialkylketones preferably having 3 to 20 carbon atoms such as dimethyl ketone ((CH₃)₂CO···); carbonamides preferably having 1 to 20 carbon atom; thiocarbonamides preferably having 1 to 20 carbon atom; thioureas preferably having 1 to 20 carbon atom; isothioureas preferably having 1 to 20 carbon atom; halogen atoms; water; etc. Incidentally, "···" represents a coordinate bond.

*X* is preferably a halogen atom or a ligand that coordinates to the transition metal atom via an isothiocyanate group, an isocyanate group, a cyanate group and/or a thiocyanate group, particularly preferably a ligand that coordinates to the transition metal atom via an isothiocyanate group and/or an isocyanate group, the most preferably a ligand that coordinates to the transition metal atom via an isothiocyanate group.

### (3) Counter Ion CI

In the general formula (IV), *CI* represents a counter ion optionally comprised in the ruthenium complex dye to neutralize charge thereof. Whether the ruthenium complex dye is a cation or an anion without the counter ion, and whether the ruthenium complex dye has net ionic charge or not without the counter ion depend on the transition metal atom, the ligand(s) and the substituent(s) thereof. When the ruthenium complex dye has a substituent containing dissociative group or atom, it may dissociate from the substituent so that the ruthenium complex dye has charge. In such a case, the charge of the entire ruthenium complex dye is neutralized by *CI*.

Typical examples of a positive counter ion represented by *CI* include: inorganic and organic ammonium ions such as tetralkyl ammonium ions and a pyridinium ion; and alkali metal ions. A negative counter ion represented by *CI* may be inorganic or organic, and examples thereof include: halide ions such as a fluoride ion, a chloride ion, a bromide ion and an iodide ion; substituted aryl sulfonate ions such as a *p*-toluene sulfonate ion and a *p*-chlorobenzene sulfonate ion; aryl disulfonate ions such as a 1,3-benzene disulfonate ion, a 1,5-naphthalene disulfonate ion and a 2,6-naphthalene disulfonate ion; alkyl sulfate ions such as a methyl sulfate ion; a sulfate ion; a thiocyanate ion; a perchlorate ion; a tetrafluoroborate ion; a hexafluorophosphate ion; a picrate ion; an acetate ion; a trifluoromethane sulfonate ion; etc. Further, the counter ion represented by *CI* may be a charge-balancing counter ion such as an ionic polymer and another dye having an opposite charge to the ruthenium complex dye comprising the ligand of the general formula (I), or a ruthenium complex ion such as bisbenzene-1,2-dithiolato nickel (III).

### (C) Specific Examples of Ruthenium Complex Dye

Specific examples *D-1* to *D-27* of the metal complex dye comprising the ligand of the general formula (I) will be illustrated below without intention of restricting the scope of the present invention defined by the claims attached hereto. Incidentally, a proton contained in the specific examples *D-1* to *D-27* is optionally dissociated.

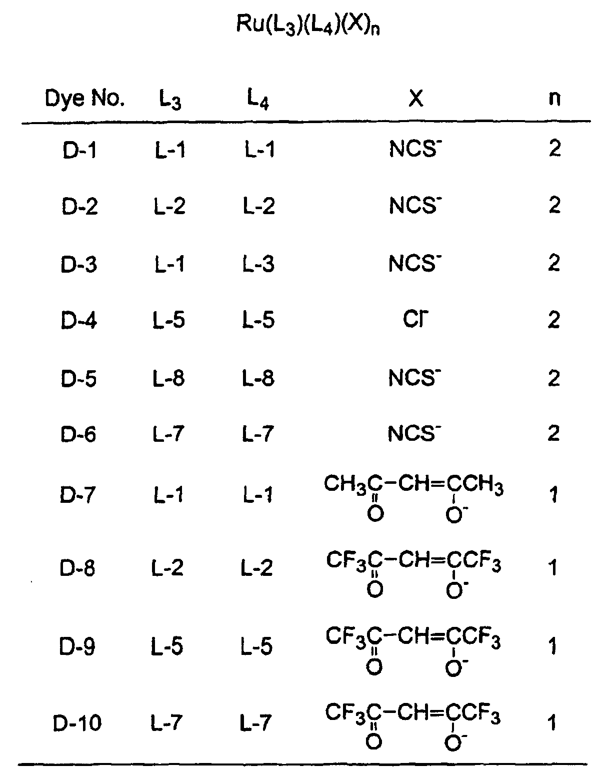

| Ru(L₅)(Xₙ) • Cl | | | | |
|---|---|---|---|---|
| Dye No. | L₅ | X | n | Cl |
| D-11 | L-11 | NCS⁻ | 3 | ⁺N(C₄H₉)₄ |
| D-12 | L-12 | NCS⁻ | 3 | ⁺N(C₄H₉)₄ |
| D-13 | L-13 | NCS⁻ | 3 | ⁺N(C₄H₉)₄ |
| D-14 | L-16 | Cl⁻ | 3 | ⁺N(C₄H₉)₄ |

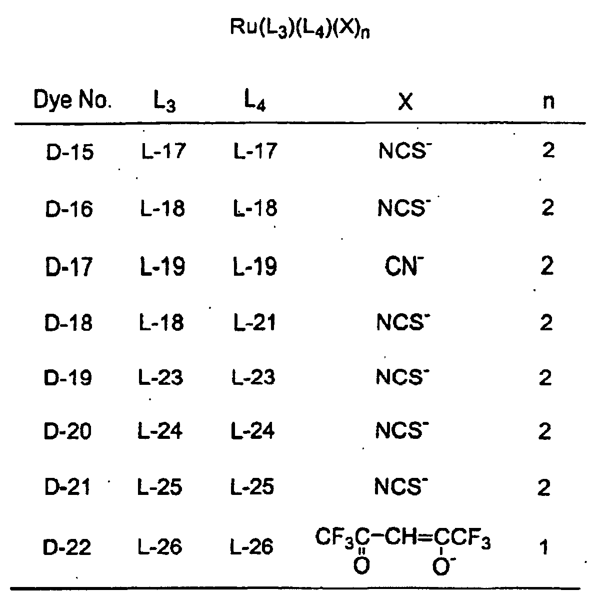

The metal complex dye used in the present invention comprising the ligand represented by the general formula (I) may be synthesized by a known general method, for example, by a method described in Inorganic Chemistry, 37, 5251 (1998), etc. The metal complex dye may be used in combination with a known dye such as a known ruthenium complex dye, a known organic dye, etc.

### [2] Photoelectric Conversion Device

A photoelectric conversion device of the present invention comprises a semiconductor fine particle sensitized by the above-mentioned ruthenium complex dye.

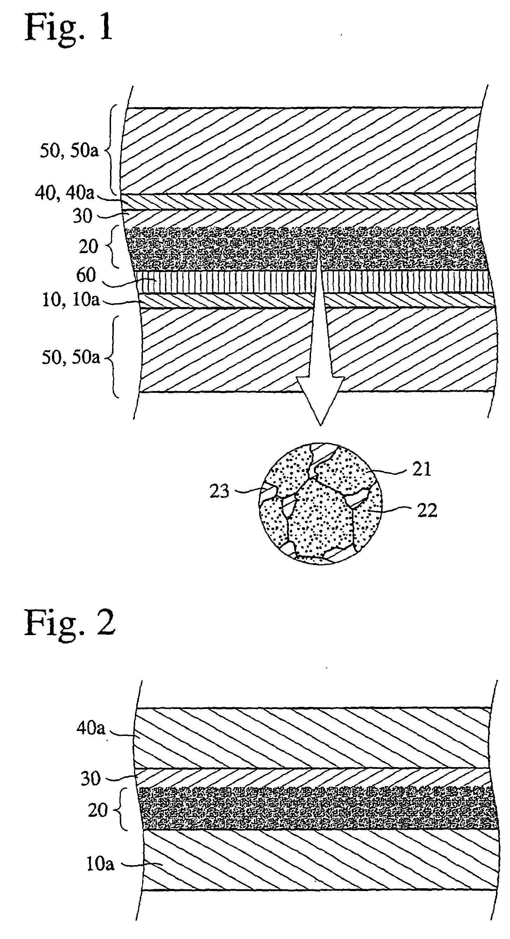

As shown in Fig. 1, the photoelectric conversion device of the present invention preferably comprises: an electrically conductive layer 10; a photosensitive layer 20 containing semiconductor fine particles 21 sensitized by dyes 22 and an charge-transporting material 23 penetrated into voids among the particles; a charge transfer layer 30; and a counter electrically conductive layer 40 laminated in this order. An undercoating layer 60 may be disposed between the electrically conductive layer 10 and the photosensitive layer 20. The charge-transporting material 23 is generally the same as the material used for the charge transfer layer 30. On the electrically conductive layer 10 and/or the counter electrically conductive layer 40 may be disposed a substrate 50 to improve the strength of the photoelectric conversion device. In the present invention, a layer composed of the electrically conductive layer 10 and the substrate 50 disposed thereon if necessary is referred to as "conductive support", and a layer composed of the counter electrically conductive layer 40 and the substrate 50 disposed thereon if necessary is referred to as "counter electrode". Incidentally, the electrically conductive layer 10, the counter electrically conductive layer 40 and the substrate 50 shown in Fig. 1 may be a transparent electrically conductive layer 10a, a transparent counter electrically conductive layer 40a and a transparent substrate 50a, respectively.

A photoelectric cell is constituted by connecting the photoelectric conversion device to an external circuit to electrically work or generate electricity in the external circuit. A photosensor is such a photoelectric conversion device as sensing optical information. Such a photoelectric cell that has the charge transfer layer composed of ion conductive material is referred to as a photo-electrochemical cell. A photoelectric cell intended for power generation using solar light is referred to as a solar cell.

In the photoelectric conversion device shown in Fig. 1, in the case of using an n-type semiconductor fine particle, a light injected to the photosensitive layer 20 excites the dye 22, etc., excited high energy electrons therein are transported to a conduction band of the semiconductor fine particles 21, and they are diffused to reach to the electrically conductive layer 10. At this time, the dye 22 is in oxidized form. In a photoelectric cell composed of the photoelectric conversion device, electrons in the electrically conductive layer 10 are returned to the oxidized dye through the counter electrically conductive layer 40 and the charge transfer layer 30 while working in the external circuit, so that the dye 22 is regenerated. The photosensitive layer 20 generally acts as a negative electrode or a photoanode, and the counter electrically conductive layer 40 generally acts as a positive electrode. In a boundary of each layer such as a boundary between the electrically conductive layer 10 and the photosensitive layer 20, a boundary between the photosensitive layer 20 and the charge transfer layer 30, a boundary between the charge transfer layer 30 and the counter electrically conductive layer 40, etc., components of each layer may be diffused and mixed.

Each layer comprised in the photoelectric conversion device of the present invention will be explained in detail below.

### (A) Conductive Support

The conductive support is composed of (1) a single layer of the electrically conductive layer, or (2) two layers of the electrically conductive layer and the substrate. In the case of (1), the electrically conductive layer is preferably made of a material that has a sufficient strength and that can sufficiently seal the photoelectric conversion device, for example, a metal such as platinum, gold, silver, copper, zinc, titanium, aluminum and an alloy composed thereof. In the case of (2), the substrate on which the electrically conductive layer containing an electrically conductive material is disposed at the photosensitive layer side may be used as the conductive support. Preferable examples of the electrically conductive material include: metals such as platinum, gold, silver, copper, zinc, titanium, aluminum, indium and alloys composed thereof; carbon; electrically conductive metal oxides such as indium-tin composite oxides and tin oxides doped with fluorine or antimony; etc. The electrically conductive layer preferably has a thickness of 0.02 to 10 µm.

The surface resistance of the conductive support is desirable as low as possible. The surface resistance is preferably 50 Ω/square or less, more preferably 20 Ω/square or less.

When light is irradiated from the conductive support side, it is preferred that the conductive support is substantially transparent. Herein, the term "substantially transparent" means that the light transmittance is 10 % or more to a light in visible region to near infrared region (400 to 1200 nm). The light transmittance is preferably 50 % or more, more preferably 80 % or more. The conductive support particularly preferably has high light transmittance to a light that the photosensitive layer has sensitivity to.

The transparent conductive support is preferably constituted by disposing the transparent electrically conductive layer of an electrically conductive metal oxide on the transparent substrate of such material as a glass and a plastic by means of coating or vapor deposition. The transparent electrically conductive layer is preferably made of tin dioxide doped with fluorine or antimony, or indium-tin oxide (ITO). The transparent substrate may be made of a glass such as low-cost soda glass excellent in strength and non-alkali glass that is not affected by alkaline elution. Additionally, a transparent polymer film is preferably used as the transparent substrate. Used as the materials for the transparent polymer film may be tetracetylcellulose (TAC), polyethylene terephthalate (PET), polyethylene naphthalate (PEN), syndiotactic polystyrene (SPS), polyphenylenesulfide (PPS), polycarbonate (PC), polyarylate (PAr), polysulfone (PSF), polyestersulfone (PES), polyimide (PI), polyetherimide (PEI), cyclic polyolefin, brominated phenoxy, etc. To secure a sufficient transparency, the coating amount of the electrically conductive metal oxide is preferably 0.01 to 100 g per 1 m² of the glass or plastic substrate.

It is preferable that a metal lead is used to reduce the resistance of the transparent conductive support. The metal lead is preferably made of a metal such as platinum, gold, nickel, titanium, aluminum, copper, silver, etc. It is preferable that the metal lead is provided on the transparent substrate by a vapor deposition method, a sputtering method, etc., the transparent electrically conductive layer of conductive tin oxide or ITO being disposed thereon. The reduction in incident light quantity owing to the metal lead is suppressed to preferably 10 % or less, more preferably 1 to 5 %.

### (B) Photosensitive Layer

The photosensitive layer contains the semiconductor fine particle on which the dye is adsorbed. In the photosensitive layer, the semiconductor fine particle acts as a photosensitive substance to absorb a light and conduct charge separation, thereby generating electrons and positive holes. With respect to the dye-sensitized semiconductor fine particle, the light absorption and the generation of the electrons and the positive holes are primarily caused in the dye, and the semiconductor fine particle receives and conveys the electrons or the positive holes.

### (1) Semiconductor

Used as the semiconductor may be: an elementary substance semiconductor such as silicon and germanium; a III-V series compound semiconductor; a metal chalcogenide such as a metal oxide, a metal sulfide, a metal selenide and a composite thereof; a compound having a perovskite structure such as strontium titanate, calcium titanate, sodium titanate, barium titanate and potassium niobate; etc. An *n*-type semiconductor is preferably used in the present invention, in which conductor electrons act as a carrier under photo-excitation condition to provide anode current.

Preferable examples of the metal chalcogenide include: oxide of titanium, tin, zinc, iron, tungsten, zirconium, hafnium, strontium, indium, cerium, yttrium, lanthanum, vanadium, niobium or tantalum; sulfide of cadmium, zinc, lead, silver, antimony or bismuth; selenide of cadmium or lead; cadmium telluride; etc. Additionally, the other compound semiconductors such as phosphides of zinc, gallium, indium or cadmium, selenides of gallium-arsenic or copper-indium, copper-indium sulfide, etc. may be used in this invention. Further, composite semiconductors such as *M*_{*x*}*O*_{*y*}*S*_{*z*} and *M*_{*1x*}*M*_{*2y*}*O*_{*z*} are also preferably used in the present invention, wherein *M, M*_{*1*} and *M*_{*2*} independently represent a metal atom, *O* represents an oxygen atom, *S* represents a sulfur atom, and *x, y* and *z* represent numbers combined with each other to form a neutral molecule.

Preferable specific examples of the semiconductor include Si, TiO₂, SnO₂, Fe₂O₃, WO₃, ZnO, Nb₂O₅, CdS, ZnS, PbS, Bi₂S₃, CdSe, CdTe, SrTiO₃, GaP, InP, GaAs, CuInS₂ and CuInSe₂. Of these semiconductors, more preferred are TiO₂, Fe₂O₃, WO₃, ZnO, Nb₂O₅, CdS, PbS, CdSe, SrTiO₃, InP, GaAs, CuInS₂ and CuInSe₂, particularly preferred are TiO₂ and Nb₂O₅, and the most preferred is TiO₂. TiO₂ use in the present invention contains anatase-type crystal structure of preferably 70 volume % or more, particularly preferably 100 volume %. It is preferable that the semiconductor is doped with a metal to increase electron conductivity thereof. This metal is preferably divalent or trivalent. Further, the semiconductor is preferably doped with a monovalent metal to prevent a reverse current from being provided from the semiconductor to the charge transfer layer.

The semiconductor may have a single crystal or poly crystal structure. The poly crystal semiconductor is preferred from the viewpoints of the production cost, the security of the raw materials, the energy-payback time, etc. The photosensitive layer is particularly preferably a porous semiconductor fine particle layer. The photosensitive layer may partly contain an amorphous semiconductor.

The particle size of the semiconductor fine particle is generally in the nm to µm level. The mean size of primary semiconductor particles, which is obtained from a diameter of a circle equivalent to a projected area thereof, is preferably 5 to 200 nm, more preferably 8 to 100 nm. Further, the mean size of secondary semiconductor particles in dispersion is preferably 0.01 to 30 µm.

Two or more of the semiconductor fine particles having a different particle size distribution may be mixed to use for the photosensitive layer. In this case, the average particle size of the smaller particles is preferably 25 nm or less, more preferably 10 nm or less. To improve a light-capturing rate of the photoelectric conversion device by scattering ray of incident light, the semiconductor fine particles having a large particle size, e.g. approximately 100 to 300 nm in diameter, may be used for the photosensitive layer.

Two or more kinds of the semiconductor fine particles may be used for the photosensitive layer. In this case, it is preferable that one is TiO₂, ZnO, Nb₂O₅ or SrTiO₃ and the other is SnO₂, Fe₂O₃ or WO₃. More preferred combination is ZnO and SnO₂, ZnO and WO₃, ZnO, SnO₂ and WO₃, etc. Each of the semiconductor fine particles may have a different diameter. Particularly preferred is a combination of TiO₂, ZnO, Nb₂O₅ or SrTiO₃ having a larger diameter and SnO₂, Fe₂O₃ or WO₃ having a smaller diameter. The larger diameter is preferably 100 nm or more, and the smaller diameter is preferably 15 nm or less.

Preferred as a method for producing the semiconductor fine particles are: sol-gel methods described in Sumio Sakka, "Zoru-Geru-Ho No Kagaku (Science of Sol-Gel Method)", Agune Shofusha (1998), Technical information Association, "Zoru-Geru-Ho Niyoru Hakumaku Coating Gijutu (Thin Film-Coating Technology by Sol-Gel Method)" (1995), etc.; and gel-sol methods described in Tadao Sugimoto, "Shin-Goseiho Geru-Zoru-Ho Niyoru Tanbunsanryusi No Gosei To Saizu-Keitaiseigyo (Synthesis of Mono-Dispersion Particles and Control of Their Size and Form by Novel Gel-Sol Method)", and MATERIA, Vol. 35, No. 9, Page 1012 to 1018 (1996). The method developed by Degussa Company, which comprises preparing oxides by subjecting chlorides to a high temperature hydrolysis in an oxyhydrogen salt, is also preferred.

In the case of using titanium oxide as the semiconductor fine particles, any of the above-described sol-gel methods, gel-sol methods and high temperature hydrolysis method are preferably used, further, a sulfuric acid method and a chlorine method described in Manabu Seino, "Sanka-Chitan Bussei To Ouyougijutu (Titanium oxide - Properties and Applied Technique)", Gihodo Shuppan, (1997) may be used. Of the sol-gel methods, also preferred are such that described in Christophe J. Barb'e, et al, Journal of American Ceramic Society, Vol. 80, No. 12, Page 3157 to 3171 (1997) and Bumside, et al, Chemistry of Materials, Vol. 10, No. 9, Page 2419 to 2425.

### (2) Semiconductor Fine Particle Layer

The semiconductor fine particles may be applied onto the conductive support by: a method where the conductive support is coated with a dispersion or a colloidal solution containing the particles; the above-mentioned sol-gel method; etc. A wet type film production method is relatively advantageous for the mass production of the photoelectric conversion device, improvement of properties of the semiconductor fine particle solution, and improvement of the adaptability of the conductive support, etc. As such a wet type film production method, coating methods, printing methods, electrolytic deposition methods and electrodeposition techniques are typical examples. Further, the semiconductor fine particle layer may be disposed by: oxidizing a metal; an LPD method where a metal solution is subjected to ligand exchange, etc.; a sputtering method; a vapor deposition method; a CVD method; or an SPD method where a thermal decomposition-type metal oxide precursor is sprayed on a heated substrate to generate a metal oxide.

The dispersion containing the semiconductor fine particles may be prepared by: the sol-gel methods mentioned above; crushing the semiconductor in a mortar; dispersing the semiconductor while grinding it in a mill; synthesizing and precipitating the semiconductor fine particles in a solvent; etc.

As a dispersion solvent, water or organic solvents such as methanol, ethanol, isopropyl alcohol, citronellol, terpineol, dichloromethane, acetone, acetonitrile, ethyl acetate, etc. may be used. A polymer such as polyethylene glycol, hydroxyethylcellulose and carboxymethylcellulose, a surfactant, an acid, a chelating agent, etc. may be used as a dispersing agent, if necessary. In particular, it is preferable that polyethylene glycol is added to the dispersion because the viscosity of the dispersion and the porosity of the semiconductor fine particle layer can be controlled by changing the molecular weight of the polyethylene glycol, and the semiconductor fine particle layer containing polyethylene glycol is hardly peeled off.

Preferred coating methods include: a roller method and a dip method as an application series; an air-knife method and a blade method as a metering series; etc. Further, preferable as a method where an application and metering can be performed at the same time are a wire-bar method disclosed in JP-B-58-4589, a slide-hopper method described in United States Patent Nos. 2,681,294, 2,761,419,2,761,791, etc., an extrusion method, a curtain method, etc. Furthermore, as for a wide use, a spin method and a spray method are preferred. As a wet type printing method, three major printing methods of a relief printing, an offset printing and a gravure printing, an intaglio printing, a gum printing, a screen printing, etc. are preferred. A preferable film production method may be selected from these methods in accordance with the viscosity of the dispersion and the desired wet thickness.

The semiconductor fine particle layer is not limited to a single layer. The dispersions each comprising the semiconductor fine particles having a different particle size may be subjected to a multi-layer coating. Further, the dispersions each containing different kinds of semiconductor fine particles, binder or additives may be subjected to a multi-layer coating. The multi-layer coating is also effectively used when the thickness of the layer is insufficient by coating of once.

Generally, when the thickness of the semiconductor fine particle layer, equal to the thickness of the photosensitive layer, becomes thicker, the amount of the dye incorporated therein per unit of the projected area increases to make the light capturing rate higher. However, because diffusion distances of the generated electrons are increased in this case, loss owing to recombination of the electric charges is also increased. Consequently, the preferable thickness of the semiconductor fine particle layer is 0.1 to 100 µm. In the case of the photoelectric cell, the thickness of the semiconductor fine particle layer is preferably 1 to 30 µm, more preferably 2 to 25 µm. A coating amount of the semiconductor fine particles per 1 m² of the substrate is preferably 0.5 to 100 g, more preferably 3 to 50 g.

After applying the semiconductor fine particles onto the conductive support, the particles are preferably subjected to a heat treatment, to electronically contact them with each other and to increase the coating strength and the adherence thereof with the support. The heating temperature is preferably 40 to 700 °C, more preferably 100 to 600 °C. The heating time is preferably 10 minutes to 10 hours. It is not preferred that the substrate having low melting point or softening point such as a polymer film is subjected to a high temperature treatment because such a substrate tends to be deteriorated thereby. The heat treatment is preferably carried out at a temperature as low as possible, for example, 50 to 350 °C, also from the viewpoint of cost. The semiconductor fine particle layer containing the smaller semiconductor fine particles having a size of 5 nm or less, a mineral acid, a metal oxide precursor, etc. can be heat-treated at such a low temperature. Further, the heat treatment may be carried out while applying ultraviolet ray, infrared ray, microwave, electric field, ultrasonic wave, etc. to the semiconductor fine particles, to reduce the heating temperature. To remove unnecessary organic compounds, etc., the heat treatment is preferably carried out in combination with evacuation, oxygen plasma treatment, washing by pure water, a solvent or a gas, etc.

After the heat treatment, the semiconductor fine particle layer may be subjected to a chemical metal-plating using an titanium tetrachloride aqueous solution, etc. or an electrochemical metal-plating using an titanium trichloride aqueous solution, etc., to increase the surface area of the semiconductor fine particles, or to enhance a purity of the particles, thereby improving the electron-injecting efficiency into the particles from the dye. Further, to prevent a reverse current from being provided from the semiconductor fine particles to the charge transfer layer, on the semiconductor fine particles is preferably adsorbed an organic compound having low electron conductivity. The organic compound preferably has a hydrophobic group.

It is preferable that the semiconductor fine particle layer has a large surface area to adsorb lots of dyes. The surface area of the semiconductor fine particle layer is preferably 10 times or more, more preferably 100 times or more of its projected area. The highest limit, even though it is not limited in particular, is generally a level of 1000 times.

### (3) Adsorption of Dye to Semiconductor Fine Particle

The dye may be adsorbed to the semiconductor fine particles by soaking the conductive support having the well-dried semiconductor fine particle layer in a dye adsorption solution, or by applying the dye adsorption solution to the semiconductor fine particle layer. In the former case, a soaking method, a dipping method, a roller method, an air-knife method, etc. may be used. In the soaking method, the dye may be adsorbed at a room temperature, or under reflux while heating as described in Japanese Patent Laid-Open No. 7-249790. As an applying method of the latter case, a wire-bar method, a slide-hopper method, an extrusion method, a curtain method, a spin method, a spray method, etc. may be used. Further, the dye may be applied to the semiconductor fine particle layer by an ink-jet method into an image, thereby providing a photoelectric conversion surface having a shape of the image.

Preferred examples of a solvent for the dye adsorption solution include: alcohols such as methanol, ethanol, *t*-butanol and benzyl alcohol; nitrile compounds such as acetonitrile, propionitrile and 3-methoxypropionitrile; nitromethane; halogenated hydrocarbons such as dichloromethane, dichloroethane, chloroform and chlorobenzene; ethers such as diethylether and tetrahydrofuran; dimethylsulfoxide; amides such as *N,N*-dimethylformamide and *N,N*-dimethylacetamide; *N*-methylpyrrolidone; 1,3-dimethylimidazolidinone; 3-methyloxazolidinone; esters such as ethyl acetate and butyl acetate; carbonates such as diethyl carbonate, ethylene carbonate and propylene carbonate; ketones such as acetone, 2-butanone and cyclohexanone; hydrocarbons such as hexane, petroleum ether, benzene and toluene; and mixtures thereof.

The total amount of the dye is preferably 0.01 to 100 mmol per the unit surface area (1 m²) of the conductive support. The amount of the dye adsorbed on the semiconductor fine particles is preferably 0.01 to 1 mmol per 1 g of the semiconductor fine particles. Such an adsorption amount of the dye effects a sufficient sensitization to the semiconductors. Too small amount of the dye results in insufficient sensitization effect. On the other hand, if the amount of the dye is excessive, the dye unadsorbed on the semiconductor fine particles floats to reduce the sensitization effect.

To increase the adsorption amount of the dye, it is preferable that the semiconductor fine particle layer is subjected to a heat treatment before the dye is adsorbed thereon. After the heat treatment, it is preferable that the dye is quickly adsorbed on the semiconductor fine particle layer having a temperature of 60 to 150 °C before the layer is cooled to the room temperature, to prevent water from adsorbing onto the semiconductor fine particle layer.

To weaken an interaction between the dyes such as association, a colorless compound may be co-adsorbed onto the semiconductor fine particles together with the dye. The colorless compound preferably has surface activity, and examples thereof include steroid compounds having a carboxyl group such as chenodeoxycholic acid, and sulfonates shown below.

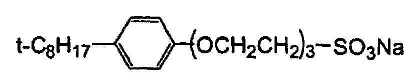

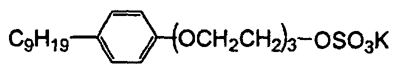

The dye unadsorbed on the semiconductor fine particle layer is preferably removed by washing immediately after the dye adsorption process. The washing is preferably carried out by a wet-type washing bath with a polar solvent such as acetonitrile or an organic solvent such as an alcohol solvent. The surface of the semiconductor fine particles may be treated with an amine compound or a quaternary salt after the dye adsorption process. The amine compound is preferably pyridine, 4-*t*-butylpyridine, polyvinylpyridine, etc., and the quaternary salt is preferably tetrabutylammonium iodide, tetrahexylammonium iodide, etc. The amine compound and the quaternary salt may be used singly when it is liquid, and may be used in a fashion of a solution in an organic solvent.

### (C) Charge Transfer Layer

The charge transfer layer replenishes electrons to the oxidized dye. The charge transfer layer may be composed of (i) an ion conductive electrolyte composition or (ii) a carrier-mediated, charge-transporting material utilizing charge transport mediated by a carrier in solid. Examples of (i) the ion conductive electrolyte composition include: molten salt electrolyte compositions containing a redox couple; electrolysis solutions where a redox couple is dissolved in a solvent; so-called gel electrolyte compositions where a solution including a redox couple is penetrated into a polymer matrix; solid electrolyte compositions; etc. Examples of (ii) the carrier-mediated, charge-transporting material include electron-transporting materials and hole-transporting materials. These materials may be used in combination with each other.

### (1) Molten Salt Electrolyte Composition

The molten salt electrolyte compositions are particularly preferably used for the charge transfer layer from the viewpoint of improving the durability and the photoelectric conversion efficiency of the photoelectric conversion device. The molten salt electrolyte composition comprises a molten salt electrolyte having a low melting point. In the present invention, pyridinium salts, imidazolium salts, triazolium salts, etc. disclosed in WO 95/18456, Japanese Patent Laid-Open No. 8-259543, "Denki Kagaku (Electrochemistry)", 65, 11, 923 (1997), etc. may be used as the molten salt electrolyte. The molten salt electrolyte preferably has the melting point of 100 °C or less, and it is particularly preferably liquid at the room temperature.

The molten salt electrolytes represented by any of the following general formulae *(Y-a), (Y-b)* and *(Y-c)* can be preferably used in the present invention.

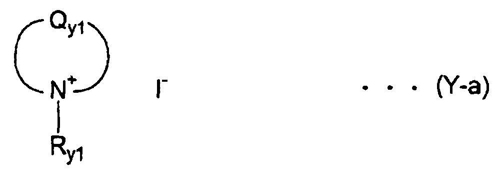

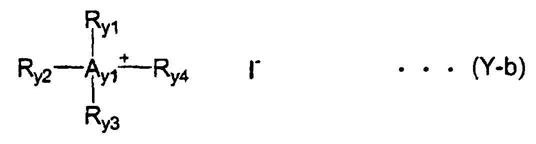

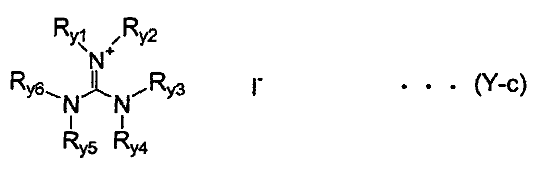

In the general formula *(Y-a), Q*_{*y1*} represents an atomic group forming an aromatic cation having a 5- or 6-membered ring structure with the nitrogen atom. *Q*_{*y1*} is preferably composed of atoms selected from the group consisting of carbon, hydrogen, nitrogen, oxygen and sulfur atoms. The 5-membered ring formed by *Q*_{*y1*} is preferably oxazole ring, thiazole ring, imidazole ring, pyrazole ring, *iso*-oxazole ring, thiadiazole ring, oxadiazole ring, triazole ring, indole ring or pyrrole ring, more preferably oxazole ring, thiazole ring or imidazole ring, particularly preferably oxazole ring or imidazole ring. The 6-membered ring formed by *Q*_{*y1*} is preferably pyridine ring, pyrimidine ring, pyridazine ring, pyrazine ring or triazine ring, more preferably pyridine ring.

In the general formula *(Y-b), A*_{*y1*} represents a nitrogen atom or a phosphorus atom.

*R*_{*y1*} to *R*_{*y6*} in the general formulae *(Y-a), (Y-b)* and *(Y-c)* independently represent: a substituted or unsubstituted alkyl group preferably having 1 to 24 carbon atom, which may be straight, branched or cyclic, such as methyl group, ethyl group, propyl group, isopropyl group, pentyl group, hexyl group, octyl group, 2-ethylhexyl group, *t*-octyl group, decyl group, dodecyl group, tetradecyl group, 2-hexyldecyl group, octadecyl group, cyclohexyl group, cyclopentyl group, etc.; or a substituted or unsubstituted alkenyl group preferably having 2 to 24 carbon atoms, which may be straight or branched, such as vinyl group, allyl group, etc. *R*_{*y1*} to *R*_{*y6*} is more preferably an alkyl group having 2 to 18 carbon atoms or an alkenyl group having 2 to 18 carbon atoms, particularly preferably an alkyl group having 2 to 6 carbon atoms, respectively.

Two or more of *R*_{*y1*} to *R*_{*y4*} in the general formula *(Y-b)* may be bonded together to form a non-aromatic ring containing *A*_{*y1*}. Two or more *of R*_{*y1*} to *R*_{*y6*} in the general formula *(Y-c)* may be bonded together to form a ring.

*Q*_{*y1*} and *R*_{*y1*} to *R*_{*y6*} in the general formulae *(Y-a), (Y-b)* and *(Y-c)* may have a substituent, respectively. Preferable examples of the substituent include: halogen atoms such as F, Cl, Br and I; cyano group; alkoxy groups such as methoxy group, ethoxy group, methoxyethoxy group and methoxyethoxyethoxy group; aryloxy groups such as phenoxy group; alkylthio groups such as methylthio group and ethylthio group; alkoxycarbonyl groups such as ethoxycarbonyl group; carbonate groups such as ethoxycarbonyloxy group; acyl groups such as acetyl group, propionyl group and benzoyl group; sulfonyl groups such as methane sulfonyl group and benzene sulfonyl group; acyloxy groups such as acetoxy group and benzoyloxy group; sulfonyloxy groups such as methane sulfonyloxy group and toluene sulfonyloxy group; phosphonyl groups such as a diethylphosphonyl group; amido groups such as acetylamino group and benzoylamino group; carbamoyl groups such as *N,N*-dimethylcarbamoyl group; alkyl groups such as methyl group, ethyl group, propyl group, isopropyl group, cyclopropyl group, butyl group, 2-carboxyethyl group and benzyl group; aryl groups such as phenyl group and toluyl group; heterocyclic groups such as pyridyl group, imidazolyl group and furanyl group; alkenyl groups such as vinyl group and 1-propenyl group; silyl groups; silyloxy groups; etc.

The molten salt electrolytes represented by the general formula *(Y-a), (Y-b)* or *(Y-c)* may form an oligomer or a polymer through *Q*_{*y1*} or *R*_{*y1*} to *R*_{*y6*}*.* The molten salt electrolytes may be used singly or in combination with each other. Further, I⁻ of the molten salt electrolytes represented by the general formula (*Y-a), (Y-b)* or *(Y-c)* may be replaced into the other anion. The other anion is preferably a halide ion such as Cl⁻ and Br⁻, SCN⁻, BF₄⁻, PF₆⁻, ClO₄⁻, N⁻(SO₂CF₃)₂, N⁻(SO₂CF₂CF₃)₂, CH₃SO₃⁻, CF₃SO₃⁻, CF₃COO⁻, BPh₄⁻, C⁻(SO₂CF₃)₃, etc., more preferably SCN⁻, BF₄⁻, N⁻(SO₂CF₃)₂, CF₃SO₃⁻ or CF₃COO⁻. Also, the molten salt electrolyte represented by the general formula *(Y-a), (Y-b)* or *(Y-c)* may be used in combination with another iodine salt such as LiI or an alkali metal salt such as CF₃COOLi, CF₃COONa, LiSCN and NaSCN. Weight ratio of the alkali metal salt is preferably 0.02 to 2 weight %, more preferably 0.1 to 1 weight %, to 100 weight % of the molten salt electrolyte composition.

The molten salt electrolytes preferably used in the present invention will be illustrated below without intention of restricting the scope of the present invention defined by the claims attached hereto.

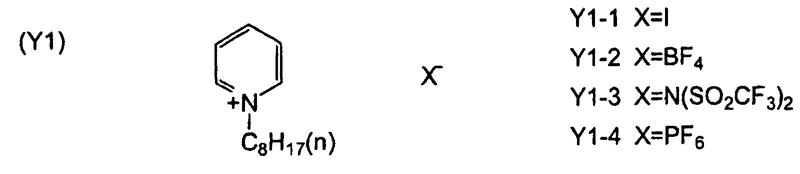

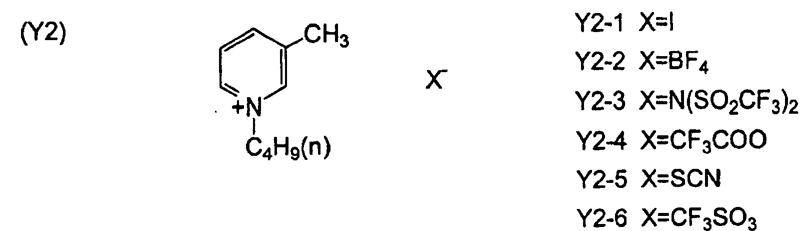

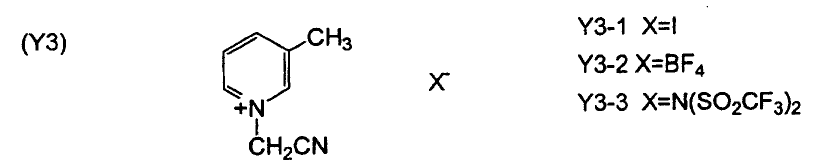

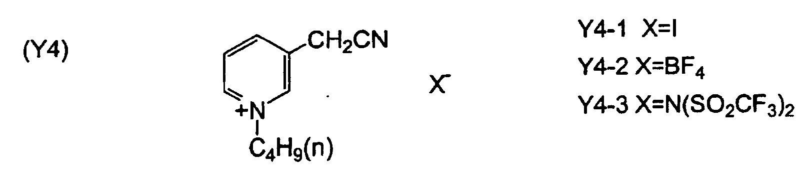

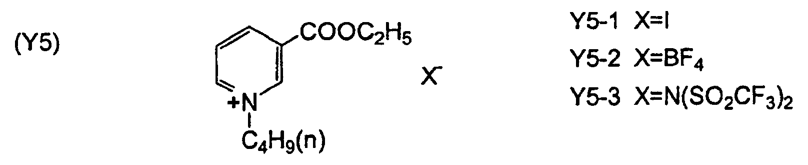

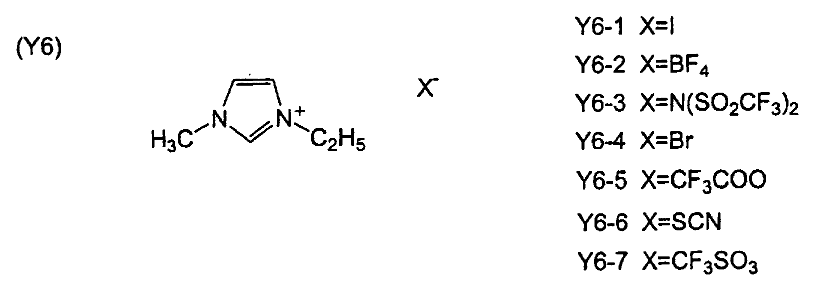

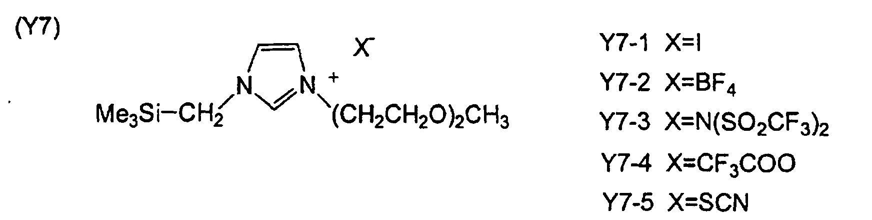

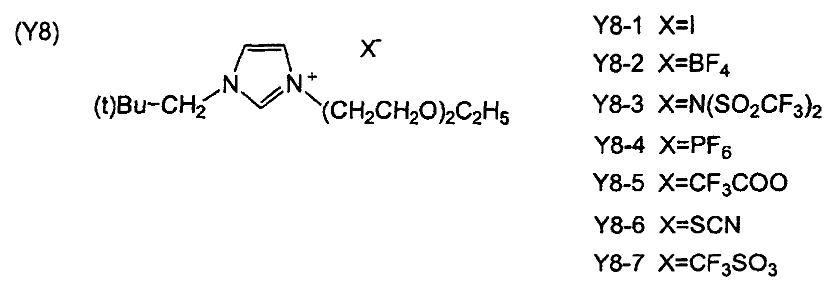

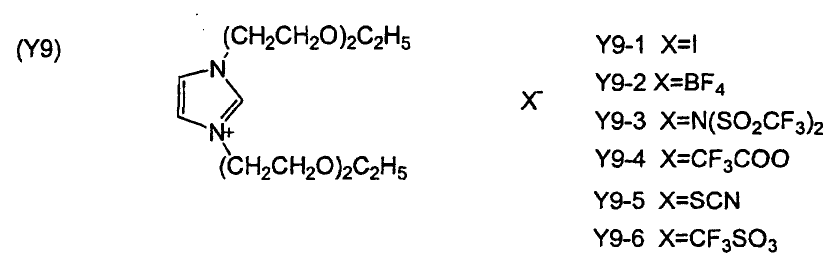

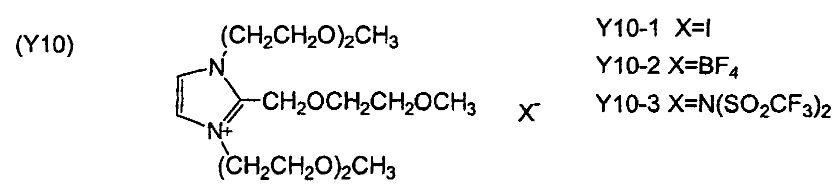

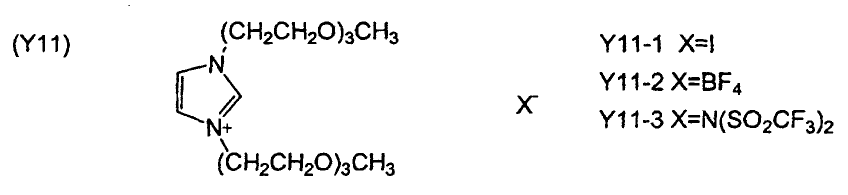

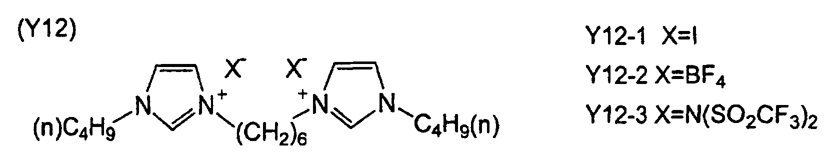

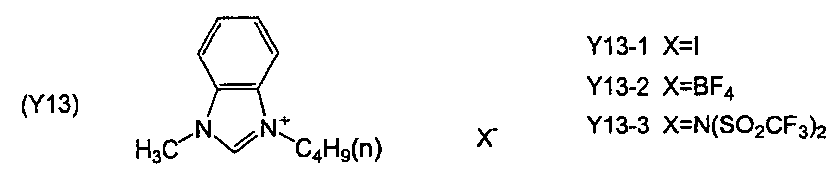

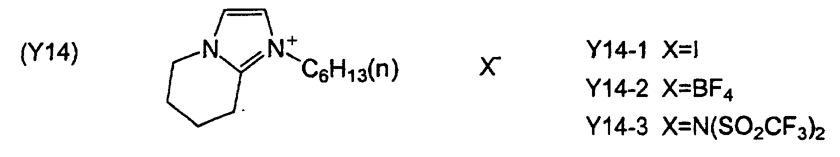

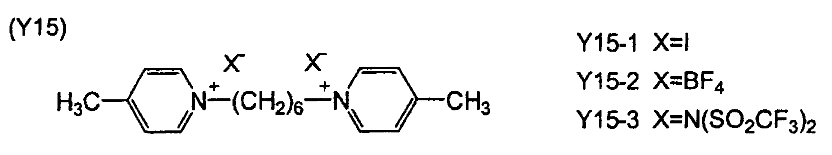

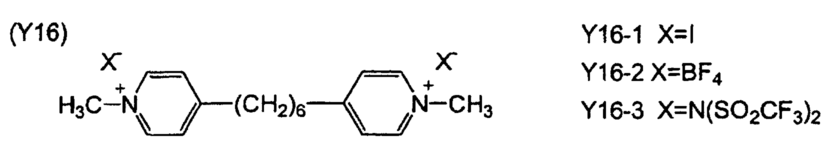

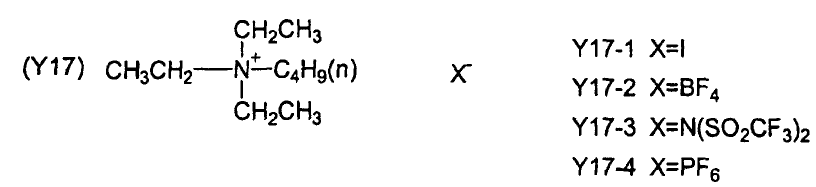

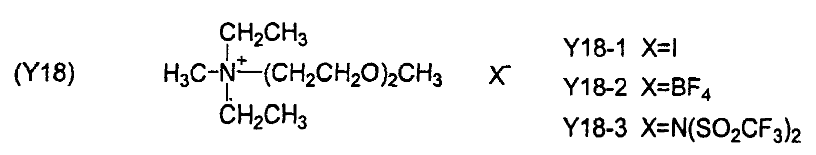

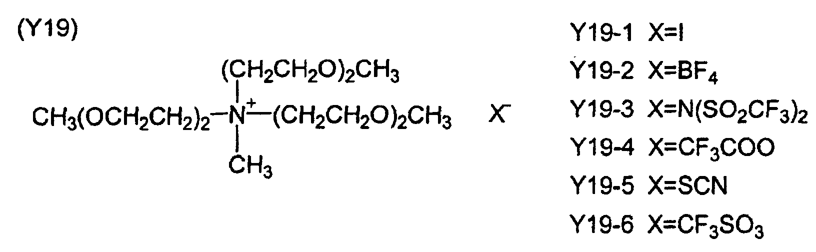

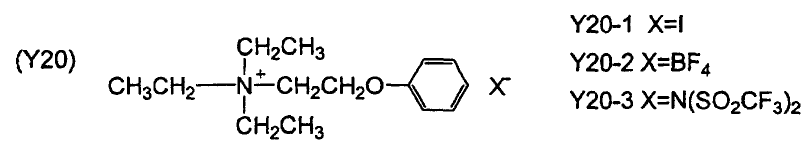

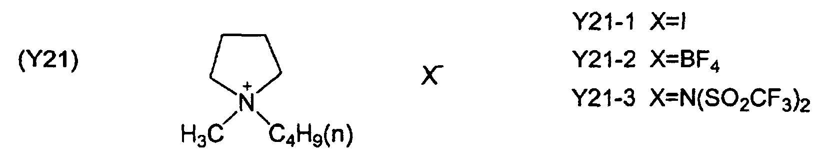

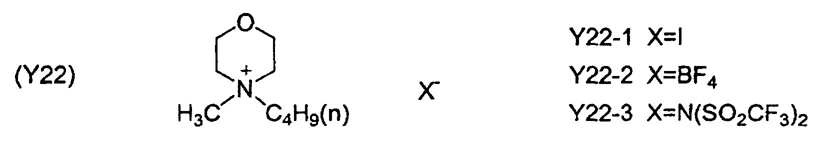

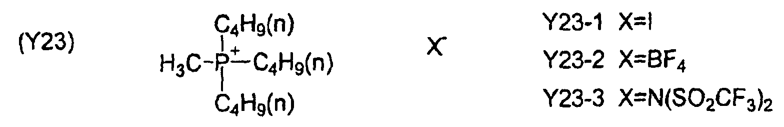

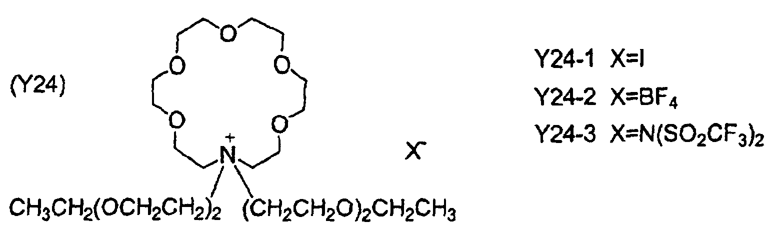

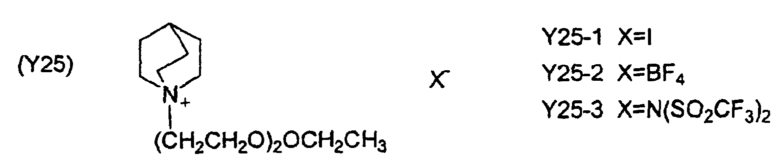

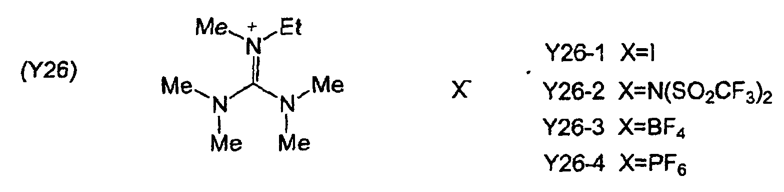

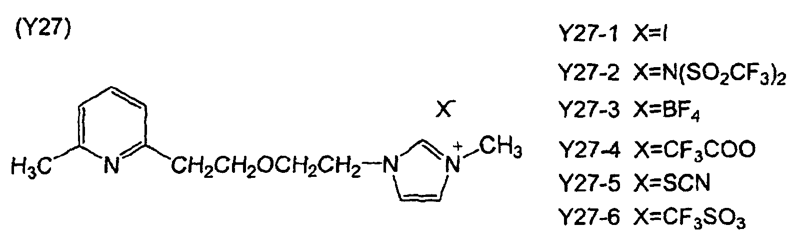

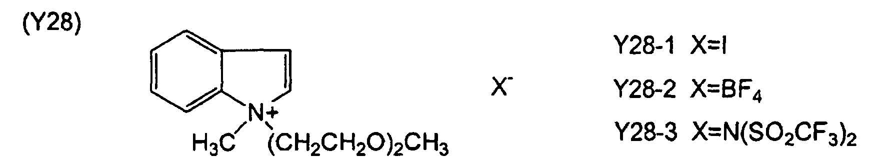

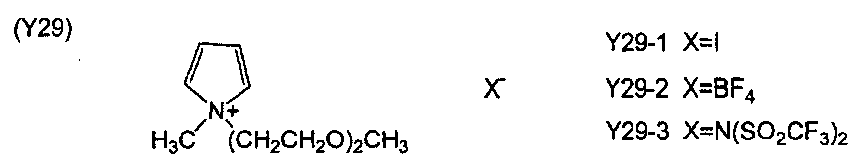

Though the molten salt electrolyte composition may comprise a solvent described below, it particularly preferably comprises no solvent. The content of the molten salt electrolyte is preferably 50 weight % or more, particularly preferably 90 weight % or more, based on the entire composition. The weight ratio of the iodine salts contained in the molten salt electrolyte composition is preferably 50 weight % or more to the entire salts contained therein.

The molten salt electrolyte composition preferably comprises iodine. The iodine-content is preferably 0.1 to 20 weight %, more preferably 0.5 to 5 weight % based on the entire composition.

### (2) Electrolysis Solution

The electrolysis solution used in the present invention is preferably composed of an electrolyte, a solvent and an additive. The electrolyte may be: a combination of I₂ and an iodide (a metal iodide such as LiI, NaI, KI, CsI and Cal₂, a quaternary ammonium iodide such as a tetralkylammonium iodide, pyridinium iodide and imidazolium iodide, etc.); a combination of Br₂ and a bromide (a metal bromide such as LiBr, NaBr, KBr, CsBr and CaBr₂, a quaternary ammonium bromide such as a tetralkylammonium bromide and pyridinium bromide, etc.); a metal complex such as a ferrocyanide-ferricyanide and a ferrocene-ferricinium ion; a sulfur compound such as sodium polysulfide and alkylthiol-alkyldisulfide; a viologen dye; hydroquinone-quinone; etc. Among them, preferred is a combination of I₂ and LiI or the quaternary ammonium iodide. A plurality of the electrolytes may be mixed to be in use.

The concentration of the electrolyte in the electrolysis solution is preferably 0.1 to 10 M, more preferably 0.2 to 4 M. Further, the electrolysis solution may comprise iodine, and the concentration of iodine therein is preferably 0.01 to 0.5 M.

The solvent used for the electrolysis solution is preferably such that has a low viscosity and a high ionic mobility, or that has a high permittivity and can increase the actual carrier concentration of the electrolysis solution, to exhibit an excellent ionic conductibility. Examples of the solvent include: carbonates such as ethylene carbonate and propylene carbonate; heterocyclic compounds such as 3-methyl-2-oxazolidinone; ethers such as dioxan and diethyl ether; chain ethers such as ethyleneglycol dialkylethers, propyleneglycol dialkylethers, polyethyleneglycol dialkylethers and polypropyleneglycol dialkylethers; alcohols such as methanol, ethanol, ethyleneglycol monoalkylethers, propyleneglycol monoalkylethers, polyethyleneglycol monoalkylethers and polypropyleneglycol monoalkylethers; glycols such as ethylene glycol, propylene glycol, polyethylene glycol, polypropylene glycol and glycerin; nitrile compounds such as acetonitrile, glutarodinitrile, methoxyacetonitrile, propionitrile and benzonitrile; aprotic polar solvents such as dimethylsulfoxide (DMSO) and sulfolane; water; etc. These solvents may be used in combination with each other.

Further, to the above-mentioned molten salt electrolyte composition and the electrolysis solution is preferably added a basic compound such as *t*-butylpyridine, 2-picoline, 2,6-lutidine, etc., as described in J. Am. Ceram. Soc., 80 (12), 3157 to 3171 (1997). The concentration of the basic compound therein is preferably 0.05 to 2 M.

### (3) Gel Electrolyte Composition

The molten salt electrolyte composition and the electrolysis solution mentioned above may be gelled or solidified to prepare the gel electrolyte composition. Gelation may be achieved by: adding a polymer; adding an oil-gelling agent; polymerization of monomers including a multifunctional monomer; a cross-linking reaction of a polymer; etc.

In the case where the gel electrolyte composition is prepared by adding a polymer, compounds described in "Polymer Electrolyte Reviews 1 and 2", edited by J. R. MacCallum and C. A. Vincent, ELSEIVER APPLIED SCIENCE may be used as the polymer. Of the compounds, polyacrylonitrile and poly(vinylidene fluoride) are preferred.

In the case where the gel electrolyte composition is prepared by adding an oil-gelling agent, compounds described in J. Chem. Soc. Japan, Ind. Chem. Soc., 46, 779 (1943), J. Am. Chem. Soc., 111, 5542 (1989), J. Chem. Soc., Chem. Commun., 390 (1993), Angew. Chem. Int. Ed. Engl., 35, 1949 (1996), Chem. Lett., 885 (1996), J. Chem. Soc., Chem. Commun., 545 (1997), etc. may be used as the oil-gelling agent. Of the compounds, preferred are those having an amide structure.

In the case where the gel electrolyte composition is prepared by a cross-linking reaction of a polymer, it is preferable that a polymer containing a group having cross-linking reactivity is used in combination with a cross-linking agent. The group having the cross-linking reactivity is preferably amino group or a nitrogen-containing heterocyclic group such as pyridyl group, imidazolyl group, thiazolyl group, oxazolyl group, triazolyl group, morpholyl group, piperidyl group, piperazyl group, etc. The cross-linking agent is preferably an electrophilic agent having a plurality of functional group that can be attacked by the nitrogen atom, for example, multi-functional alkyl halides, aralkyl halides, sulfonates, acid anhydrides, acyl chlorides, isocyanates, α,β-unsaturated sulfonyl compounds, α,β-unsaturated carbonyl compounds, α,β-unsaturated nitrile compounds, etc. Cross-linking methods disclosed in Japanese Patent Laid-Open Nos. 2000-17076 and 2000-86724 may be used in the present invention.

Further, a method for gelling an electrolysis solution disclosed in Japanese Patent Laid-Open No. 11-185863 and a method for gelling a molten salt electrolyte composition disclosed in Japanese Patent Laid-Open No. 2000-58140 may be used in this invention.

### (4) Hole-Transporting Material

In the present invention, an organic solid hole-transporting material, an inorganic solid hole-transporting material or a combination thereof may be used for the charge transfer layer instead of the ion conductive electrolyte composition such as the molten salt electrolyte composition.

### (a) Organic Hole-Transporting Material

Preferred examples of the organic hole-transporting material used in this invention include: aromatic amines disclosed in J. Hagen, et al., Synthetic Metal, 89, 215 to 220 (1997), Nature, Vol. 395, 8 Oct. 1998, Page 583 to 585, WO 97/10617, United States Patent Nos. 4,923,774 and 4,764,625, Japanese Patent Laid-Open Nos. 59-194393, 5-234681, 4-308688, 3-269084, 4-129271, 4-175395, 4-264189, 4-290851, 4-364153, 5-25473, 5-239455, 5-320634,6-1972, 7-138562, 7-252474 and 11-144773, etc.; triphenylenes disclosed in Japanese Patent Laid-Open Nos. 11-149821, 11-148067 and 11-176489, etc.; oligothiophene compounds disclosed in Adv. Mater., 9, No. 7, 557, 1997, Angew. Chem. Int. Ed. Engl., 34, 3, 303 to 307, 1995, J. Am. Chem. Soc., Vol. 120, No. 4, Page 664 to 672, 1998, etc.; and conductive polymers such as polypyrrole disclosed in K. Murakoshi, et al., Chem. Lett., 471, 1997 and polyacetylene, poly(*p*-phenylene), poly(*p*-phenylenevinylene), polythienylenevinylene, polythiophene, polyaniline, polytoluidine and derivatives thereof disclosed in "Handbook of Organic Conductive Molecules and Polymers", Vols. 1 to 4, edited by NALWA, published by WILEY.

As described in Nature, Vol. 395, 8 Oct. 583 to 585 (1998), to the organic hole-transporting material may be added a compound having cation radical such as tris(4-bromophenyl)aminium hexachloroantimonate to control the dopant level, or a salt such as Li[(CF₃SO₂)₂N] to achieve potential-control of surface of the semiconductor, thereby compensating a space-charge layer.

### (b) Inorganic Hole-Transporting Material

The inorganic hole-transporting material may be a *p*-type inorganic compound semiconductor. Band gap of the *p-*type inorganic compound semiconductor is preferably 2 eV or more, more preferably 2.5 eV or more. Ionization potential of the *p*-type inorganic compound semiconductor should be smaller than that of the photosensitive layer to reduce holes of the dye. Although the ionization potential of the *p-*type inorganic compound semiconductor may be selected depending on the kind of the dye, generally, it is preferably 4.5 to 5.5 eV, more preferably 4.7 to 5.3 eV. The *p*-type inorganic compound semiconductor is preferably a compound having a monovalent copper such as CuI, CuSCN, CuInSe₂, Cu(In,Ga)Se₂, CuGaSe₂, Cu₂O, CuS, CuGaS₂, CuInS₂, CuAlSe₂, etc. Among them, CuI and CuSCN are preferred, and CuI is the most preferred. GaP, NiO, CoO, FeO, Bi₂O₃, MoO₂, Cr₂O₃, etc. are also used as the *p*-type inorganic compound semiconductor.

### (5) Method for Forming Charge Transfer Layer

The charge transfer layer may be provided by any of the following two methods. One is a method where the counter electrode is stuck on the photosensitive layer beforehand and the material for the charge transfer layer in the liquid state is made to penetrate a gap therebetween. Another is a method where the charge transfer layer is directly disposed on the photosensitive layer, the counter electrode being then disposed thereon.

In the former method, the material for the charge transfer layer may be made to penetrate the gap by a normal pressure process utilizing capillarity, or by a reduced pressure process where the material is pumped up from the gap to replace gas phase therein with liquid phase.

In the case of providing a wet charge transfer layer by the latter method, the wet charge transfer layer is applied to the photosensitive layer, the counter electrode is disposed on the wet charge transfer layer without drying it, and edges thereof is subjected to a treatment for preventing liquid-leakage, if necessary. In the case of providing a gel charge transfer layer by the latter method, the charge transfer material may be applied in the liquid state and gelled by polymerization, etc. In this case, the counter electrode may be disposed on the charge transfer layer before or after drying and fixing the charge transfer layer.

The charge transfer layer composed of the electrolysis solution, the wet organic hole-transporting material, the gel electrolyte composition, etc. may be disposed by a method, a roller method, a dip method, an air-knife method, an extrusion method, a slide-hopper method, a wire-bar method, a spin method, a spray method, a cast method, various printing methods, etc. similarly to the case of forming the semiconductor fine particle layer, or adsorbing a dye to the semiconductor mentioned above.

The charge transfer layer composed of the solid electrolyte, the solid hole transporting material, etc. may be formed by a dry film-forming method such as a vacuum deposition method and a CVD method, and followed by disposing the counter electrode thereon. The organic hole-transporting material may be made to penetrate into the photosensitive layer by a vacuum deposition method, a cast method, a coating method, a spin-coating method, a soaking method, an electrolytic polymerization method, a photo-polymerization method, etc. The inorganic hole-transporting material may be made to penetrate into the photosensitive layer by a cast method, a coating method, a spin-coating method, a soaking method, an electrolytic deposition method, an electroless deposition method, etc.

### (D) Counter Electrode

The counter electrode is the counter electrically conductive layer, which is supported by the substrate, if necessary. Examples of the electrically conductive material used for the counter electrically conductive layer include: metals such as platinum, gold, silver, copper, aluminum, magnesium and indium; carbon; and electrically conductive metal oxides such as indium-tin composite oxides and fluorine-doped tin oxides. Among them, preferred are platinum, gold, silver, copper, aluminum and magnesium. The substrate of the counter electrode is preferably made of a glass or a plastic to be coated or vapor-deposited with the electrically conductive material. The counter electrically conductive layer preferably has a thickness of 3 nm to 10 µm, although the thickness is not particularly limited. The surface resistance of the counter electrically conductive layer is desirably as low as possible. The surface resistance is preferably 50 Ω/square or less, more preferably 20 Ω/square or less.

Light may be irradiated from any one or both side of the conductive support and the counter electrode, so that at least one of them should be substantially transparent to have light reached to the photosensitive layer. From a viewpoint of improving electric generation efficiency, it is preferable that the conductive support is substantially transparent to irradiate light therethrough. In this case, the counter electrode preferably has a light-reflective property. Such a counter electrode may be composed of a glass or a plastic having a vapor-deposited layer of metal or electrically conductive oxide, or metal thin film.

The counter electrode may be disposed by applying, metal-plating or vapor-depositing (PVD, CVD, etc.) the electrically conductive material directly onto the charge transfer layer. It is preferable that the metal lead is used to reduce the resistance of the counter electrode, as is similar to the conductive support. The metal lead is particularly preferably used for the transparent counter electrode. Preferable embodiments of the metal lead used for the counter electrode are the same as those of the metal lead used for the conductive support mentioned above.

### (E) Others

It is preferable that a fine semiconductor thin film is provided between the conductive support and the photosensitive layer as an undercoating layer to prevent short-circuit of the counter electrode and the conductive support, particularly in the case of the charge transfer layer composed of the electron-transporting material or the hole-transporting material. The undercoating layer is preferably made TiO₂, SnO₂, Fe₂O₃, WO₃, ZnO or Nb₂O₅, more preferably made of TiO₂. The undercoating layer may be disposed by a spray-pyrolysis method described in Electrochim. Acta, 40, 643 to 652 (1995), a sputtering method, etc. The thickness of the undercoating layer is preferably 5 to 1000 nm, more preferably 10 to 500 nm.

Functional layers such as a protective layer and a reflection-preventing layer may be disposed on any one or both of the conductive support and the counter electrode. The functional layers may be disposed by a method selected in accordance with the materials therefor, such as a coating method, a vapor-deposition method and a sticking method.

### (F) Interior Structure of Photoelectric Conversion Device

As described above, the photoelectric conversion device may have various interior structures in accordance with an end of use. The structures are classified into two major forms, a structure allowing light incidence from both faces, and a structure allowing it from only one face. Each of Figs. 2 to 9 illustrates an example of the interior structure of the photoelectric conversion device, which is preferable in the present invention.

As for the structure illustrated in Fig. 2, the photosensitive layer 20 and the charge transfer layer 30 are disposed between the transparent electrically conductive layer 10a and the transparent counter electrically conductive layer 40a. This structure allows light incidence from both faces of the device.

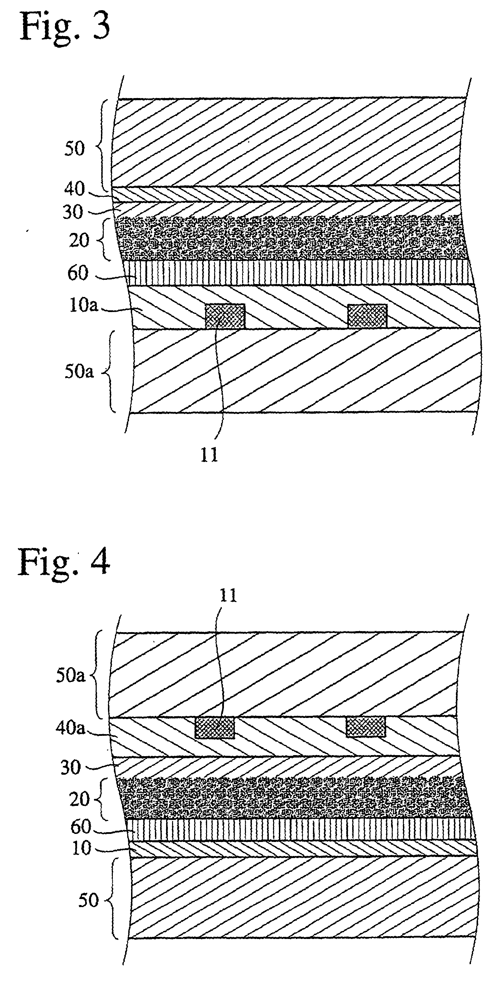

As for the structure illustrated in Fig. 3, on the transparent substrate 50a partially having the metal lead 11 is disposed the transparent electrically conductive layer 10a, the undercoating layer 60, the photosensitive layer 20, the charge transfer layer 30 and the counter electrically conductive layer 40 are laminated in this order, and the substrate 50 is further placed thereon. This structure allows light incidence from the electrically conductive layer side.

As for the structure illustrated in Fig. 4, the photosensitive layer 20 is disposed on the substrate 50 having the electrically conductive layer 10 through the undercoating layer 60, the charge transfer layer 30 and the transparent counter electrically conductive layer 40a are disposed thereon, and further the transparent substrate 50a locally having the metal lead 11 is placed on the counter electrically conductive layer 40a so that the metal lead 11 side orients inward. This structure allows light incidence from the counter electrode side.

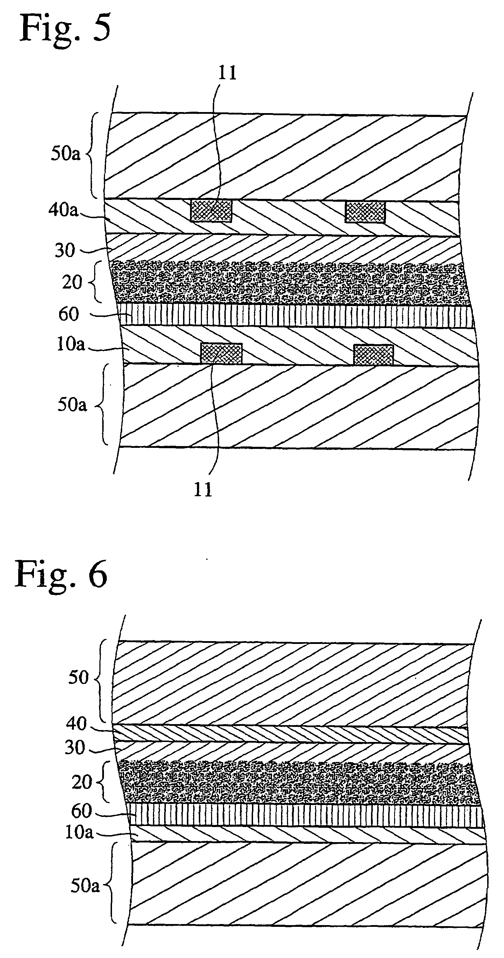

As for the structure illustrated in Fig. 5, on the two transparent substrates 50a each having the metal lead 11 partially are formed the transparent electrically conductive layer 10a and the transparent counter electrically conductive layer 40a, respectively, and the undercoating layer 60, the photosensitive layer 20 and the charge transfer layer 30 placed between the conductive layers. This structure allows light incidence from both faces of the photoelectric conversion device.

As for the structure illustrated in Fig. 6, on the transparent substrate 50a having the transparent electrically conductive layer 10a is disposed the photosensitive layer 20 through the undercoating layer 60, the charge transfer layer 30 and the counter electrically conductive layer 40 are formed thereon, and further the substrate 50 is placed on the counter electrically conductive layer 40. This structure allows light incidence from the electrically conductive layer side.

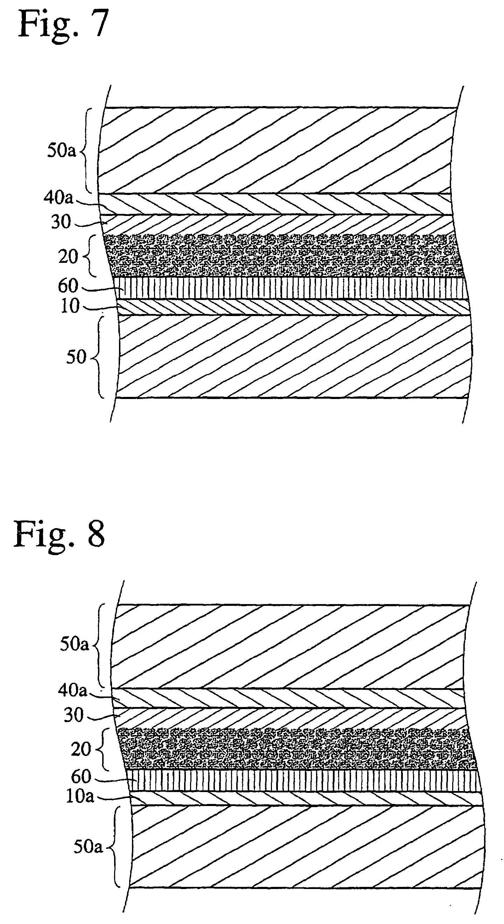

As for the structure illustrated in Fig. 7, on the substrate 50 having the electrically conductive layer 10 is disposed the photosensitive layer 20 through the undercoating layer 60, the charge transfer layer 30 and the transparent counter electrically conductive layer 40a are formed thereon, and further the transparent substrate 50a is placed on the layer 40a. This structure allows light incidence from the counter electrode side.

As for the structure illustrated in Fig. 8, on the transparent substrate 50a having the transparent electrically conductive layer 10a is disposed the photosensitive layer 20 through the undercoating layer 60, the charge transfer layer 30 and the transparent counter electrically conductive layer 40a are formed thereon, and further the transparent substrate 50a is placed on the layer 40a. This structure allows light incidence from both faces of the photoelectric conversion device.

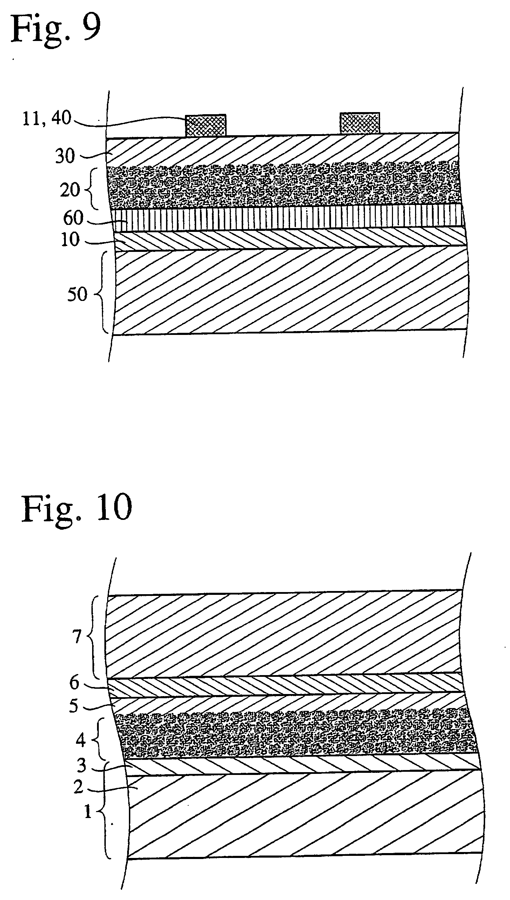

As for the structure illustrated in Fig. 9, the photosensitive layer 20 is disposed on the substrate 50 having the electrically conductive layer 10 through the undercoating layer 60, the solid charge transfer layer 30 is disposed thereon, and further the counter electrically conductive layer 40 or the metal lead 11 is locally placed on the solid charge transfer layer 30. This structure allows light incidence from the counter electrode side.

### [3] Photoelectric Cell

The photoelectric cell of the present invention is constituted by connecting the photoelectric conversion device of the present invention to an external circuit to electrically work or generate electricity in the external circuit. Such a photoelectric cell that has the charge transfer layer composed of ion conductive electrolyte composition is referred to as a photo-electrochemical cell. A photoelectric cell intended for power generation using solar light is referred to as a solar cell.

The side face of the photoelectric cell is preferably sealed with a polymer or an adhesive agent, etc. to prevent deterioration and volatility of the content in the cell. The external circuit is connected to the conductive support and the counter electrode via a lead. Various known circuits may be used in the present invention.

In the case where the photoelectric conversion device of the present invention is applied to the solar cell, the interior structure of the solar cell may be essentially the same as that of the photoelectric conversion device mentioned above. The solar cell comprising the photoelectric conversion device of the present invention may have a known module structure. In general module structure of the solar cell, cells are placed on a substrate of metal, ceramic, etc. and covered with a packing resin, a protective glass, etc., whereby light is introduced from the opposite side of the substrate. The solar cell module may have a structure where the cells are placed on a substrate of a transparent material such as a tempered glass to introduce light from the transparent substrate side. Specifically, a superstraight type module structure, a substrate type module structure, a potting type module structure, substrate-integrated type module structure that is generally used in amorphous silicon solar cells, etc. are known as the solar cell module structure. The solar cell comprising the photoelectric conversion device of the present invention may have a module structure properly selected from the above structures in accordance with ends and environment at use, and preferably has a module structure disclosed in Japanese Patent Laid-Open No. 2000-268892.

### EXAMPLES

The present invention will be explained in more detail with reference to examples below without intention of restricting the scope of the present invention.

### [A] Synthesis of Ruthenium Complex Dye

### (1) Synthesis of Ruthenium Complex Dye D-1

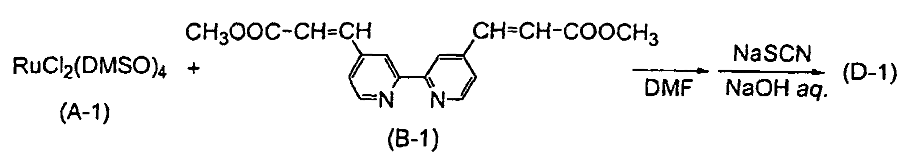

0.17 g of the compound *A-1* and 0.12 g of the compound *B-1* were dissolved in 30 ml of dimethylformamide (DMF) and refluxed under nitrogen for 6 hours. Then, 0.14 g of sodium thiocyanate and 20 ml of an aqueous solution containing 0.2 g of sodium hydroxide were added to the resulting mixture, and further refluxed for 6 hours while heating. This reaction mixture was cooled and concentrated, and the residue was purified by "Sephadex Column LH-20" (developing solvent: water) followed by neutralization, to obtain 0.20 g of the ruthenium complex dye *D-1* as crystal. Structure of the product was identified by NMR and MS spectra. Thus-obtained ruthenium complex dye *D-1* exhibited an absorption maximum in visible wavelength region at 535 nm in H₂O.

### (2) Synthesis of Ruthenium Complex Dye D-2

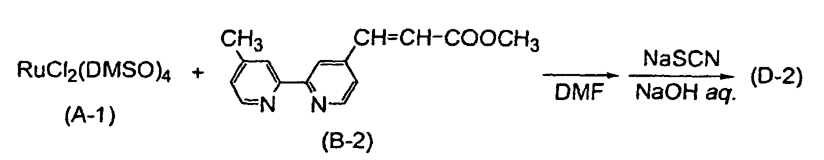

0.17 g of the compound *A-1* and 0.10 g of the compound *B-2* were dissolved in 30 ml of DMF and refluxed under nitrogen for 6 hours. Then, 0.14 g of sodium thiocyanate and 20 ml of an aqueous solution containing 0.2 g of sodium hydroxide were added to the resulting mixture, and further refluxed for 6 hours while heating. This reaction mixture was cooled and concentrated, and the residue was purified by "Sephadex Column LH-20" (developing solvent: water) followed by neutralization, to obtain 0.18 g of the ruthenium complex dye D-2 as crystal. Structure of the product was identified by NMR and MS spectra. Thus-obtained ruthenium complex dye *D-2* exhibited an absorption maximum in visible wavelength region at 517 nm in H₂O.

### (3) Synthesis of Ruthenium Complex Dye D-3

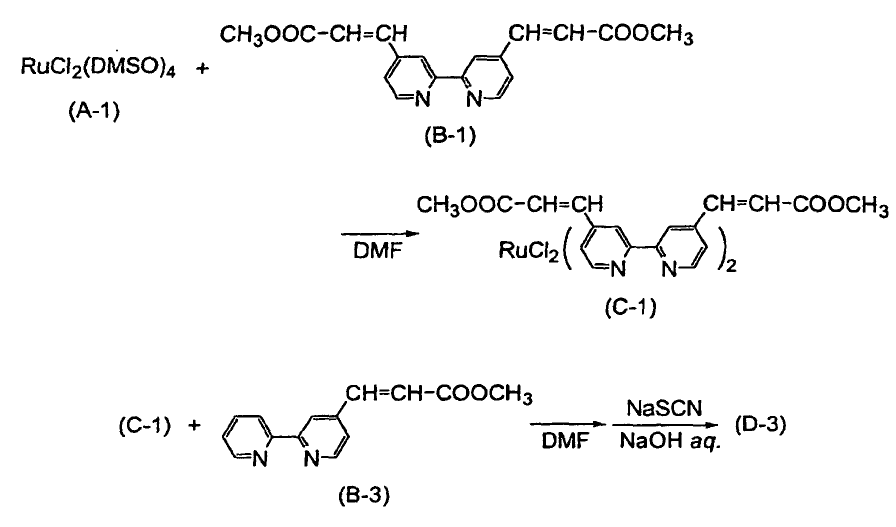

0.17 g of the compound *A-1* and 0.06 g of the compound *B-1* were dissolved in 30 ml of DMF, refluxed under nitrogen for 6 hours, cooled and concentrated to obtain crude compound *C-1.* This crude compound *C-1* and 0.06 g of the compound *B-3* were dissolved in 30 ml of DMF, refluxed under nitrogen for 6 hours. Then, 0.14 g of sodium thiocyanate and 20 ml of an aqueous solution containing 0.2 g of sodium hydroxide were added to the resulting mixture, and further refluxed for 6 hours while heating. This reaction mixture was cooled and concentrated, and the residue was purified by "Sephadex Column LH-20" (developing solvent: water) followed by neutralization, to obtain 0.05 g of the ruthenium complex dye *D-3* as crystal. Structure of the product was identified by NMR and MS spectra. Thus-obtained ruthenium complex dye *D-3* exhibited an absorption maximum in visible wavelength region at 527 nm in H₂O.

### (4) Synthesis of Ruthenium Complex Dye D-15

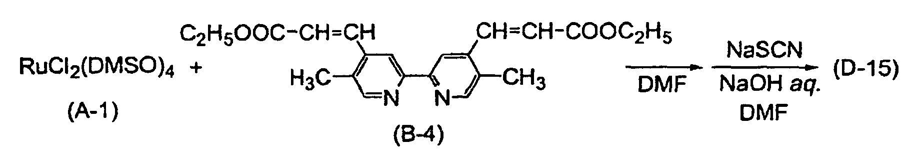

0.17 g of the compound *A-1* and 0.10 g of the compound *B-4* were dissolved in 30 ml of DMF and refluxed under nitrogen for 6 hours. Then, 0.14 g of sodium thiocyanate and 20 ml of an aqueous solution containing 0.2 g of sodium hydroxide were added to the resulting mixture, and further refluxed for 6 hours while heating. This reaction mixture was cooled and concentrated, and the residue was purified by "Sephadex Column LH-20" (developing solvent: water) followed by neutralization, to obtain 0.18 g of the ruthenium complex dye *D-15* as crystal. Structure of the product was identified by NMR and MS spectra. Thus-obtained ruthenium complex dye *D-15* exhibited an absorption maximum in visible wavelength region at 517 nm in H₂O.

Other ruthenium complex dye represented by the general formula (IV) can be synthesized by a method similar to the above-mentioned methods for synthesizing the metal complex dyes *D-1* to *D-3* and *D-15.*

### [B] Preparation of Titanium Dioxide Dispersion

15 g of titanium dioxide "Degussa P-25" manufactured by Nippon Aerosil K.K., 45 g of water, 1 g of dispersant "Triton X-100" manufactured by Aldrich, and 30 g of zirconia beads having a diameter of 0.5 mm manufactured by Nikkato K.K. was charged in a stainless steel vessel coated with Teflon inside having an inner volume of 200 ml, and subjected to a dispersion treatment for 2 hours at 1500 rpm by means of a sand-grinder mill manufactured by Imex K.K. After the dispersion treatment, the zirconia beads were removed by filtration to obtain a titanium dioxide dispersion. The titanium dioxide particles in thus-obtained dispersion had an average particle diameter of 2.5 µm, the particle diameter being measured by Master Sizer manufactured by MALVERN.

### [C] Preparation of Dye-Sensitized TiO₂ Electrode

The above titanium dioxide dispersion was applied to an electrically conductive surface of a conductive glass having a fluorine-doped tin oxide coating by a glass bar. Used as the conductive glass was "TCO Glass-U" manufactured by Asahi Glass K.K. having a surface resistance of approximately 30 Ω/square, which was cut into 20 mm × 20 mm in size beforehand. Herein, an adhesive tape was attached to the electrically conductive surface of the conductive glass at portions of from the edge to 3 mm as a spacer, eight conductive glasses were arranged such that the adhesive tapes come to both edges thereof, and to the eight conductive glasses were applied the titanium dioxide dispersion at once. The titanium dioxide dispersion-applied conductive glass was air-dried for one day at a room temperature after peeling the adhesive tape. The amount of the applied titanium dioxide (semiconductor fine particle) was 20 g/m².

Then, the conductive glass was placed in an electric furnace "muffle furnace FP-32" manufactured by Yamato Science K.K., followed by burning at 450 °C for 30 minutes to obtain a TiO₂ electrode. After the TiO₂ electrode was taken out of the electric furnace and cooled, it was immersed in a methanol solution comprising the ruthenium complex dye *D-1* for 15 hours so that the dye is adsorbed on the titanium dioxide, further immersed in 4-*t*-butylpyridine for 15 minutes, washed with ethanol and air-dried to obtain a dye-sensitized TiO₂ electrode. The concentration of the dye *D-1* in the methanol solution was 3 × 10⁻⁴ mol/l, and the thickness of thus-obtained photosensitive layer was 5 µm.

### [D] Production of Photoelectric Conversion Device

The 20 mm × 20 mm in size of the dye-sensitized TiO₂ electrode prepared as described above was put on a platinum-deposited glass having the same size. Then, an electrolysis solution of 3-methoxypropionitrile comprising 0.65 mol/l of 1-methyl-3-hexylimidazolium iodide and 0.05 mol/l of iodine was permeated into a crevice between the glasses through capillarity and introduced into the dye-sensitized TiO₂ electrode, to obtain a photoelectric conversion device *C-1*. According to this example, a photoelectric conversion device having a structure shown in Fig. 10, in which a conductive glass 1 composed of a glass 2 and an electrically conductive layer 3, a dye-sensitized TiO₂ layer 4, an electrolyte layer 5, a platinum layer 6 and a glass 7 were laminated in this order, was produced.

Photoelectric conversion devices *C-2* to *C-12* were produced in the same manner as the device *C-1* except that the ruthenium complex dyes shown in Tables 1 and 2 were used instead of the ruthenium complex dye *D-1,* respectively.

### [E] Measurement of Photoelectric Conversion Efficiency

### (1) Photoelectric Conversion Devices C-1 to C-7

Each of the photoelectric conversion devices *C-1* to *C-7* was measured with respect to the photoelectric conversion efficiency *IPCE* (incident photon to current conversion efficiency) for a monochromatic light having a wavelength of 800 nm by an *IPCE*-measuring apparatus manufactured by Optel Co. The results are shown in Table 1.

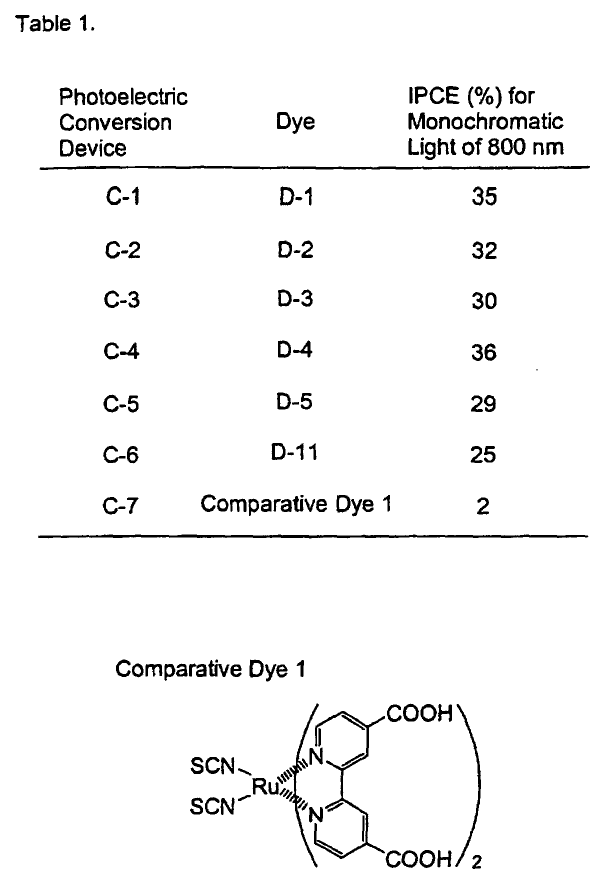

As shown in Table 1, the photoelectric conversion devices *C-1* to *C-6* of the present invention were excellent in the photoelectric conversion efficiency for the monochromatic light having a wavelength of 800 nm as compared with the photoelectric conversion device *C-7* using the known, comparative dye 1.

### (2) Photoelectric Conversion Devices C-1 and C-7 to C-12

A simulated sunlight was irradiated to each of the photoelectric conversion devices *C-1* and *C-7* to *C-12,* so that each device was measured with respect to the generated electricity by the current-voltage tester "Keithley SMU238" to obtain a photoelectric conversion efficiency (η). The results are shown in Table 2. Incidentally, the simulated sunlight was obtained by passing the light of a 500 W Xenone Lamp manufactured by Ushio K.K. through an "AM 1.5 filter" manufactured by Oriel Co. and a sharp cut filter "Kenko L-42". The simulated sunlight was free of ultraviolet rays and had intensity of 100 mW/cm².

**Table 2.**

| Photoelectric Conversion Device | Dye | Photoelectric Conversion Efficiency (%) |
|---|---|---|
| C-1 | D-1 | 8.21 |
| C-8 | D-15 | 9.42 |
| C-9 | D-17 | 7.20 |
| C-10 | D-20 | 8.91 |
| C-11 | D-11 | 6.80 |
| C-12 | D-27 | 5.52 |
| C-7 | Comparative Dye 1 | 5.30 |

As shown in Table 2, the photoelectric conversion devices *C-1* and *C-8* to *C-12* of the present invention were excellent in the photoelectric conversion efficiency as compared with the photoelectric conversion device *C-7* using the known, comparative dye 1.

As described in detail above, a photoelectric conversion device of the present invention, which comprises a semiconductor fine particle sensitized by a ruthenium complex dye having a particular ligand represented by general formula (1), can convert a light having a wavelength of 800 nm or more to electricity with an excellent photoelectric conversion efficiency. Thus, a photoelectric cell of the present invention using this photoelectric conversion device can utilize infrared light contained in solar light abundantly to be remarkably usable as a solar cell.

## Claims

1. A ruthenium complex dye represented by the following general formula (IV): 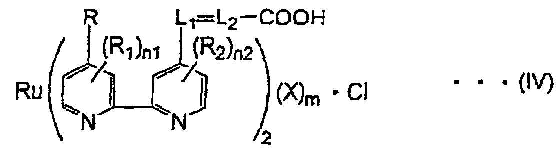 wherein R, R₁ and R₂ independently represent a substituent selected from the group consisting of alkyl groups, alkenyl groups, cycloalkyl groups, aryl groups, heterocyclic groups, alkoxy groups, a hydroxyl group, amino groups and halogen atoms;
n1 and n2 independently represent an integer of 0 to 2; R₁'s and R₂'s are the same or different groups that optionally bond together to form a ring when n1 and n2 are 2, respectively;
L₁ and L₂ independently represent a substituted or unsubstituted methine group selected from the group consisting of alkyl groups, alkenyl groups, clycloalkyl groups, aryl groups, heterocyclic groups, alkoxy groups, amino groups and halogen atoms;
X represents a monodentate or bidentate ligand that coordinates to ruthenium atom via one or two group selected from the group consisting of acyloxy groups; acylthio groups; acylaminooxy groups; thioacyloxy groups; thioacylthio groups; thiocarbonate groups; dithiocarbonate groups; trithiocarbonate groups; alkylthio groups; arylthio groups; alkoxy groups; aryloxy groups; an isothiocyanate group; an isocyanate group; a cyanate group; and a thiocyanate group;
or β-diketonato ligands; β-dithioketonato ligands; β-ketothionato ligands; β-thionketonato ligands; dialkylketones; carbonamides; thiocarbonamides; thioureas; isothioureas; halogen atoms; and water;
or a halogen atom or a ligand that coordinates to ruthenium atom via an isothiocyanate group, an isocyanate group, a cyanate group and/or a thiocyanate group; m represents 1 or 2; and
CI represents a counter ion optionally comprised to neutralize charge of said ruthenium complex dye selected from the group consisting of a positive counter ion from inorganic and organic ammonium ions or a negative counter ion from inorganic or organic ions including halide ions; substituted aryl sulfonate ions; aryl disulfonate ions; alkyl sulfate ions; a sulfate ion; a thiocyanate ion; a perchlorate ion; a tetrafluoroborate ion; a hexafluorophosphate ion; a picrate ion; an acetate ion; and a trifluoromethane sulfonate ion;
or a charge-balancing counter ion and another dye having an opposite charge to the ruthenium complex dye.

2. A photoelectric conversion device comprising a semiconductor fine particle sensitized by a ruthenium complex dye of claim 1.

3. A photoelectric cell comprising a photoelectric conversion device of claim 2.

## Patentansprüche

1. Ruthenium-Komplex-Farbstoff, dargestellt durch die folgende allgemeine Formel (IV): 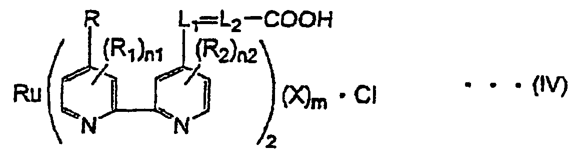 wobei R, R₁ und R₂ unabhängig einen Substituenten darstellen, der aus der Gruppe ausgewählt ist, die aus Alkylgruppen, Alkenylgruppen, Cycloalkylgruppen, Arylgruppen, heterocyclischen Gruppen, Alkoxygruppen, einer Hydroxylgruppe, Aminogruppen und Halogenatomen besteht; n1 und n2 unabhängig eine ganze Zahl von 0 bis 2 darstellen; Rᵢ's und R₂'s dieselben oder unterschiedliche Gruppen sind, welche optional miteinander eine Verbindung eingehen, um einen Ring zu bilden, wenn n1 und n2 jeweils 2 sind;
L₁ und L₂ unabhängig eine substituierte oder unsubstituierte Methingruppe darstellen, die aus der Gruppe ausgewählt ist, die aus Alkylgruppen, Alkenylgruppen, Cycloalkylgruppen, Arylgruppen, heterocyclische Gruppen, Alkoxygruppen, Aminogruppen und Halogenatomen besteht;
X einen einzahnigen oder zweizahnigen Liganden darstellt, der mit dem Rutheniumatom über eine oder zwei Gruppen koordiniert, die ausgewählt ist aus der Gruppe, die aus Acyloxygruppen, Acylthiogruppen, Acylaminooxygruppen, Thioacyloxygruppen, Thioacylthiogruppen, Thiocarbonatgruppen, Dithiocarbonatgruppen, Trithiocarbonatgruppen, Alkylthiogruppen, Arylthiogruppen, Alkoxygruppen, Aryloxygruppen, einer Isothiocyanatgruppe, einer Isocyanatgruppe, einer Cyanatgruppe und einer Thiocyanatgruppe besteht;
oder einen β-Diketonato-Liganden, β-Dithioketonato-Liganden, β-Ketothionato-Liganden, β-Thioketonato-Liganden, Di-alkylketonen, Carbonamiden, Thiocarbonamiden, Thioharnstoffen, Isothioharnstoffen, Halogenatomen und Wasser besteht;
oder ein Halogenatom oder einen Liganden, der mit dem Rutheniumatom über eine Isothiocyanatgruppe, eine Isocyanatgruppe, eine Cyanatgruppe und/oder eine Thiocyanatgruppe koordiniert, wobei m 1 oder 2 darstellt; und
CI ein Gegenion darstellt, das optional vorgesehen ist, um die Ladung des Ruthenium-Komplex-Farbstoffs zu neutralisieren, ausgewählt aus der Gruppe, die aus einem positiven Gegenion aus anorganischen und organischen Ammoniumionen oder einem negativen Gegenion aus anorganischen und organischen Ionen, enthaltend Halogenidionen, substituierten Arylsulfonationen, Aryldisulfonationen, Alkylsulfationen, einem Sulfation, einem Thiocyanation, einem Perchloration, einem Tetrafluorboration, einem Hexafluorphosphation, einem Picration, einem Acetation und einem Trifluormethansulfonation, besteht;
oder ein Ladungsausgleich-Gegenion und einem weiteren Farbstoff, der eine Ladung entgegengesetzt zu dem Ruthenium-Komplex-Farbstoff aufweist.

2. Photoelektrische Umsetzvorrichtung, aufweisend ein Halbleiter-Feinpartikel, sensibilisiert durch einen Ruthenium-Komplex-Farbstoff von Anspruch 1.

3. Photoelektrische Zelle, aufweisend eine photoelektrische Umsetzvorrichtung von Anspruch 2.

## Revendications

1. Colorant complexe de ruthénium représenté par la formule générale (IV) suivante : 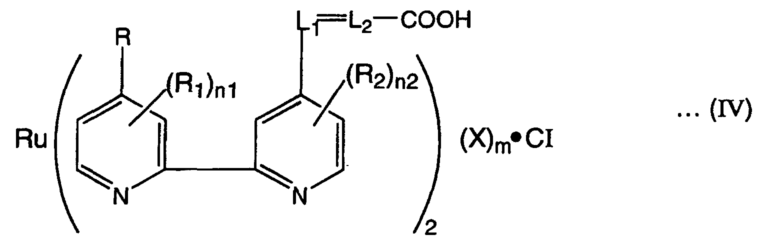 dans laquelle R, R₁ et R₂ représentent indépendamment un substituant choisi dans le groupe constitué par des groupes alkyle, des groupes alcényle, des groupes cycloalkyle, des groupes aryle, des groupes hétérocycliques, des groupes alcoxy, un groupe hydroxyle, des groupes amino et des groupes d'halogène;
n₁ et n₂ représentent indépendamment un nombre entier de 0 à 2 ; les R₁ et les R₂ sont des groupes identiques ou différents qui se lient éventuellement ensemble pour former un cycle quand n₁ et n₂ sont 2, respectivement ;
L₁ et L₂ représentent indépendamment un groupe méthine substitué ou non substitué choisi dans le groupe constitué par des groupes alkyle, des groupes alcényle, des groupes cycloalkyle, des groupes aryle, des groupes hétérocycliques, des groupes alcoxy, des groupes amino et des atomes d'halogène ;
X représente un ligand monodentate ou bidentate qui se coordonne avec l'atome de ruthénium via un ou deux groupes choisis dans le groupe constitué par des groupes acyloxy ; des groupes acylthio ; des groupes acylaminooxy ; des groupes thioacyloxy ; des groupes thioacylthio, des groupes thiocarbonate ; des groupes dithiocarbonate ; des groupes trithiocarbonate ; des groupes alkylthio ; des groupes arylthio ; des groupes alcoxy ; des groupes aryloxy ; un groupe isothiocyanate ; un groupe isocyanate ; un groupe cyanate ; et un groupe thiocyanate ;
ou des ligands β-dicétonato ; des ligands β-dithiocétonato, des ligands β-cétothionato ; des ligands β-thioncétonato ; des dialkylcétones ; des carbonamides ; des thiocarbonamides ; des thiourées ; des isothiourées ; des atomes d'halogène ; et de l'eau ;
ou un atome d'halogène ou un ligand qui se coordonne avec l'atome de ruthénium via un groupe isothiocyanate, un groupe isocyanate, un groupe cyanate et/ou un groupe thiocyanate ; m représente 1 ou 2 ; et
CI représente un contre-ion éventuellement compris pour neutraliser la charge dudit colorant complexe de ruthénium choisi dans le groupe constitué par un contre-ion positif provenant des ions ammonium inorganiques et organiques ou un contre-ion négatif provenant des ions inorganiques ou organiques comprenant des ions halogénure ; des ions arylsulfonate substitués ; des ions aryldisulfonate ; des ions alkylsulfate ; un ion sulfate, un ion thiocyanate, un ion perchlorate ; un ion tétrafluoroborate ; un ion hexafluorophosphate ; un ion picrate ; un ion acétate, et un ion trifluorométhanesulfonate ;
ou un contre-ion équilibrant les charges et un autre colorant ayant une charge opposée au colorant complexe de ruthénium.

2. Dispositif de conversion photoélectrique comprenant de fines particules semi-conductrices sensibilisées par un colorant complexe de ruthénium selon la revendication 1.

3. Cellule photoélectrique comprenant un dispositif de conversion photoélectrique selon la revendication 2.
